# EUROPEAN PATENT SPECIFICATION

(11) **EP 1 826 183 B1**
(45) Date of publication and mention of the grant of the patent: **06.01.2010**
(21) Application number: 07250765.0
(22) Date of filing: 23.02.2007
(51) Int. Cl.: C02F 1/461

(54) **Ionized water and method of producing same**
Ionisiertes Wasser und Verfahren zu dessen Herstellung
Eau ionisée et son procédé de fabrication

(30) Priority: 24.02.2006 US 776502 P
(43) Date of publication of application: 29.08.2007
(60) Divisional application: 09163420.4
(73) Proprietor: Skyview Enterprises, New York NY 10011 (US)
(72) Inventor: Skyview Enterprises, New York NY 10011 (US)
(74) Representative: Tombling, Adrian George

(56) References cited:
- EP-A- 0 826 636
- EP-A1- 0 605 882
- EP-A2- 1 293 481
- WO-A-03/042112
- WO-A-03/045446
- WO-A-03/050044
- JP-A- 7 303 885
- US-A1- 2002 027 070
- US-A1- 2002 185 380
- US-A1- 2004 094 406
- US-B1- 6 168 692
- US-B1- 6 723 226

## Description

### BACKGROUND OF THE INVENTION

### TECHNICAL FIELD

Generally, the present invention relates to the field of ionized water. More specifically, the present invention relates to a method of producing water having health promoting benefits.

### BACKGROUND ART

Generally, there are two types of commercially available ionized waters: alkaline ionized water and acidic ionized water. These waters possess several benefits for the promotion of health. Alkaline ionized water has been suggested to prevent or reverse common colds, diabetes, osteoporosis and obesity. Acidic ionized water provides benefits such the promotion of wound healing, reduction of acne, relief of throat and mouth sores, and disinfectant functions.

A third type of ionized water exists. It is neutral ionized water (NIW) and is attracting interest as a safe disinfectant agent. Based on the fact that it has anti-bacterial properties without containing any chemical compounds such as steroids or antibiotics, NIW is used as a wound healing spray for animals, and has been proven to be safe even if it is ingested or licked by animals. NIW is pH neutral and does not cause skin or eye irritation. However, little is known about the benefits of NIW in health promotion.

In order to produce NIW, electrolysis has been usually carried out in the presence of NaCl. A derivative of this electrolysis, hypochlorous acid, has been shown to be the anti-bacterial component (Rutala, W. A., et al.). However, hypochlorous acid causes inflammatory stress on mammalian cells because of its strong oxidant property (Winterbourn, C.C.).

It has been documented that reactive oxygen species (ROS) can cause many types of damage to biomolecules and cellular events, consequently resulting in the development of a variety of pathologic states such as inflammation, cancer and aging (Grisham, M. B., et al., and Lavrovsky, Y., et al.). The oldest life forms such as bacteria have developed hydrogenases, which are enzymes that catalyze reactive oxygen species resulting in the production of active hydrogen and thus neutralizing the damaging effects of reactive oxygen (Ghirardi, M. L., et al.). However, human and other evolved animals do not possess hydrogenase. In order to deactivate reactive oxygen, an anti-oxidant can be supplied from exogenous sources, such as food and water.

In an effort to eliminate the presence of harmful substances in ionized water, a variety of methods of forming ionized water have been developed. One such method disclosed in Japanese Publication 7-303885 is the use of diaphragm or non-diaphragm electrolysis to form acidic water on the side of an anode and alkali water on the side of a cathode and the inclusion of a calcium salt and a water-soluble reducing agent between the two forms of water for performing the electrolysis. The method is intended to create water containing alkali ions without forming harmful substances. The process is cumbersome and the resulting water can still include chlorine or hypochlorite.

Accordingly, there is a need for a method of producing hypochlorite-free ionized water. It would also be beneficial to create a method of producing hypochlorite-free ionized water that possesses the same properties as neutral ionized water.

Additionally, the fitness market currently provides numerous drinks designed to promote health benefits. Such drinks include numerous vitamins designed to provide the drinker with a predetermined benefit. Examples of such benefits include, but are not limited to, relaxation, more energy, and the ability to better concentrate. While such benefits are helpful, there is no evidence that the drinks provide any benefits to the overall well-being of the individual. It would therefore be beneficial to develop an ionized water that can be used not only as disclosed, but also as a drink to promote overall health for individuals.

### SUMMARY OF THE INVENTION

According to the present invention, there is provided a method of forming hydrogen enriched hypochlorite free neutral ionized water as defined in the appending claims.

### BRIEF DESCRIPTION OF THE DRAWINGS

Other advantages of the present invention will be readily appreciated, as the same becomes better understood by reference to the following detailed description when considered in connection with the accompanying drawings wherein:
Figure 1 is a bar graph demonstrating the elevated concentration of dissolved hydrogen in the NIW of the invention compared to control de-ionized unprocessed waters (DIW and 0.12% NaCl);
Figure 2 demonstrates the anti-oxidant activity of the NIW of the invention;
Figure 3A is a fluorescent microscopic demonstration of the anti-oxidant activity of NIW on the intracellular reactive oxygen species (ROS) induced by inflammatory stimulation with mitogen, LPS, in mouse osteoclast precursor RAW264.7 cells. Figure 3B depicts untreated control cells, wherein ROS was not induced in the RAW264.7 cells;
Figure 4 contains bar graphs illustrating the anti-inflammatory effect of NIW containing hypochlorite on the production of proinflammatory cytokines by human peripheral blood mononuclear cells stimulated with mitogen, peptidoglycan, *in vitro,* wherein " * " means significantly lower than control medium stimulated with peptidoglycan by Student's *t* test (P < 0.05);
Figure 5 is a chart demonstrating the lack of correlation between the concentration of hypochlorous acid in the NIW containing hypochlorite and the inhibitory effect of NIW on the production of proinflammatory cytokine, TNF-α;
Figure 6 is a graph illustrating the effect of NIW containing hypochlorite, regular non-electrolyzed de-ionized water containing dissolved hydrogen (DH-DIW) or sodium hypochlorite (NaClO) water on the production of IL-1β by human peripheral blood mononuclear cells stimulated with peptidoglycan *in vitro,* wherein " * " means significantly lower than control medium stimulated with peptidoglycan by Student's *t* test (P < 0.05) and " ** " means significantly higher than control medium stimulated with peptidoglycan by Student's *t* test (P < 0.05);
Figure 7 includes graphs illustrating the stability of hypochlorite containing NIW's anti-inflammatory activity on the production of proinflammatory cytokines by human peripheral blood mononuclear cells over time (at 1.5 months or 3 months) and with NIW generated using different concentrations of NaCl;
Figure 8 illustrates containing hypochlorite NIW's inhibitory effect on *in vitro* osteoclast differentiation in the presence or absence of RANKL (receptor activator of NF-Kappa B ligand) using the RAW264.7 mouse osteoclast precursor cell line;
Figure 9 shows containing hypochlorite NIW's inhibitory effect on osteoclast differentiation *in vitro* using RAW264.7 cells at differing concentrations of RANKL, wherein " * " means significantly lower than control DIW medium that contains a corresponding amount of RANKL, by Student's *t* test (P < 0.01);
Figure 10 demonstrates containing hypochlorite NIW's inhibitory effect on RANKL/LPS-mediated *in vivo* osteoclast induction in mouse calvaria tissue, wherein " * " means significantly elevated compared to negative control group A by Student's *t* test (P < 0.05) and " ** " means significantly lower than group B by Student's *t* test (P < 0.05);
Figure 11 includes histological demonstrations of tartrate resistant acid phosphatase (TRAP)-positive osteoclasts induced in mouse calvaria tissue, wherein Figure 11A is the calvaria tissue of a mouse injected with RANKL/LPS and maintained with DIW as drinking water *Ad libitum* and Figure 11B is calvaria tissue of a mouse injected with RANKULPS and maintained with NIW containing hypochlorite as drinking water *Ad libitum* [x 200];
Figure 12 is a bar graph showing the inhibitory effect of NIW containing hypochlorite on the IL-1β produced in the serum of RANKL/LPS-injected mice;
Figure 13 demonstrates the inhibitory effect of NIW containing hypochlorite on *in vivo* RANKL/LPS- dependent periodontal bone loss using a rat model;
Figure 14 demonstrates *in vitro* cancer cell growth inhibition by NIW containing hypochlorite using two types of cancer cell lines, MDA-MB-435S (human breast ductal carcinoma) and Jurkat E6-1 (human acute T cell leukemia), wherein " * " means significantly lower than control medium at the corresponding proportion of dilution, by Student's *t* test (P < 0.05);
Figure 15 is a graph showing the hypochlorite concentration produced in a NaCl solution after different time periods of electrolysis using the machine of the present invention;

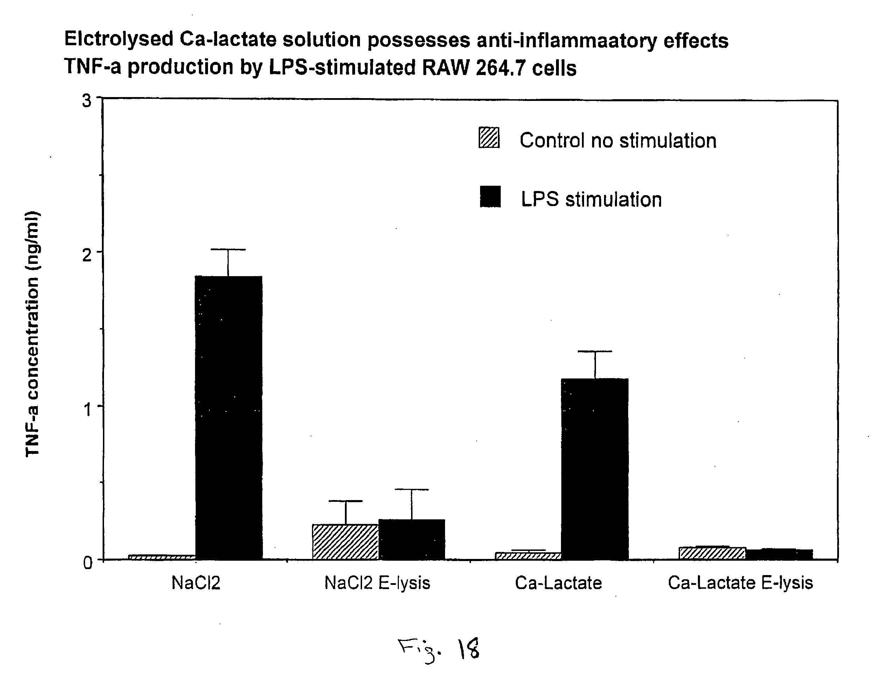

Figure 16 is a graph showing TNF-α production by RAW264.7 cells in I-W electrolyzed in the presence of NaCl at different time periods using the machine of the present invention (Fig 18-22);

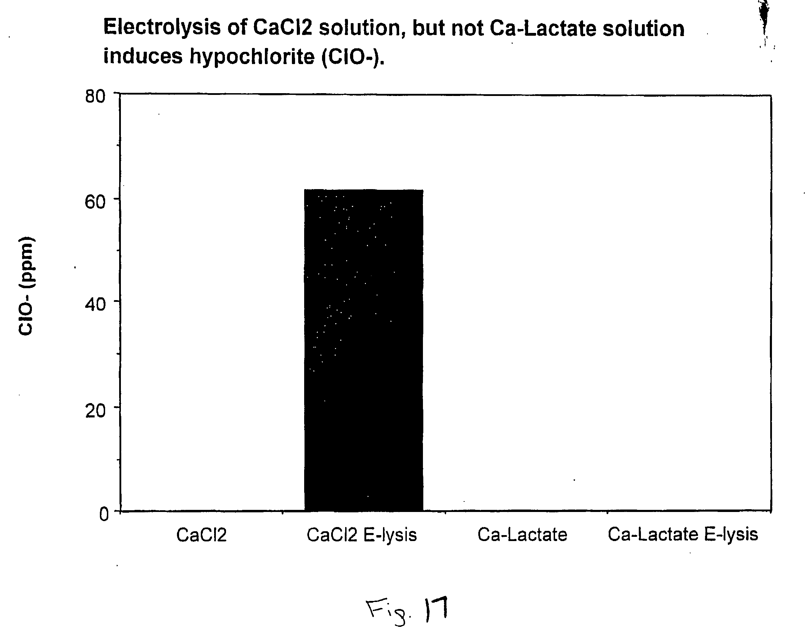

Figure 17 is a graph showing the I-W electrolyzed in the presence of CaCl₂ , using the machine of the present invention, which possesses hypochlorite wherein Ca-lactate I-W solution which was also generated using the machine of the present invention does not produce hypochlorite;
Figure 18 is a graph showing the anti-inflammatory effects of electrolyzed Ca-lactate solution on TNF-α production by LPS-stimulated RAW264.7 cells; and
Figure 19 is a photograph of a remote magnetic stirrer for use in the machine of the present invention;
Figure 20 is a photograph of a remote magnetic stirrer for use in the machine of the present invention;
Figure 21 is a photograph of a remote magnetic stirrer for use in the machine of the present invention;
Figure 22 is a photograph of a remote magnetic stirrer for use in the machine of the present invention;
Figure 23 is a graph showing the effects of the NIW containing hypochlorite on loss of body weight in mice with DDS induced colitis;

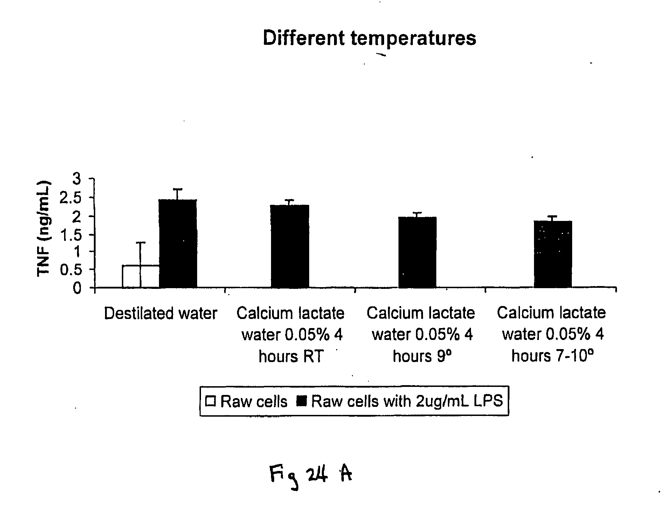

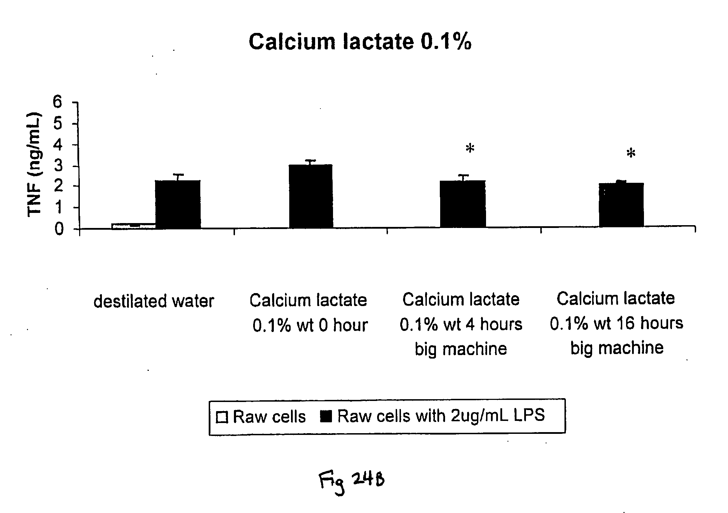

Figures 24A and 24B are graphs showing the anti-inflammatory effects of electrolyzed Ca-lactate solution using the machine of the present invention on TNF-α production by LPS-stimulated cells at varying concentrations of Ca-lactate, Figure 24A shows .05% Ca-lactate and Figure 24B shows.1% Ca-lactate;
Figures 25A and 25B are graphs showing the effects of the and NIW generated using the machine of the present invention in the presence of NaCl or NaHCO₃ respectively on the Concanavalin-A induced mouse acute hepatitis;
Figure 26 is a graph showing the effects of electrolyzed NIW containing hypochlorite on Nitric oxide production by LPS-stimulated RAW264.7 cells;
Figure 27 is a graph showing the effects of electrolyzed NIW containing hypochlorite on Osteoclast differentiation by RANKL-stimulated RAW264.7 cells;
Figure 28 is a graph showing the hydrogen production in water during the electrolysis process using the machine of the present invention wherein either NaCl or NaHCO₃ is dissolved in de-ionized water;
Figure 29 is a graph showing the anti-inflammatory effects of ionized water that was generated by electrolysis of the water in the presence of 0.05% NaHCO₃ which does not contain hypochlorite;
Figure 30 is a graph showing the hypochlorite free NIW from NaHCO₃ solution using the machine of the present invention;
Figure 31 is a graph showing the stable neutral pH in the electrolyzed water using the machine of the present invention;
Figure 32 is a graph showing the effects of temperature during the electrolysis process to generate NIW from NaCl solution using the machine of the present invention; and
Figure 33 is a graph showing the effects of temperature during the electrolysis process to generate NIW from NaHCO₃ solution using the machine of the present invention.

### DETAILED DESCRIPTION OF THE INVENTION

The present invention relates to a method of forming hydrogen enriched hypochlorite free neutral ionized water as defined in the appending claims. The ionized water has numerous health promoting benefits and thus can be used to generally promote user health or the ionized water can be used for the prevention and treatment of diseases and conditions as recited herein.

The term "ionized water" as used herein is intended to include ionized water including calcium lactate, sodium bicarbonate, or other non-hypochlorite generating compounds. The ionized water is produced using the methodology disclosed herein and can be used in treating diseases and conditions. For example, the water is processed as described herein such that it displays one or more of the following biological effects: the inhibition of proinflammatory cytokine production, the inhibition of bone resorption, and the inhibition of cancer cell growth.

The term "neutral pH" as used herein is intended to include a pH within a range of 5-9, as is known to those of skill in the art.

The term "calcium lactate" as used herein is intended to include, but is not limited to, a white crystalline salt made by the action of lactic acid on calcium having the molecular formula (CH₃CHOHCOO)₂Ca-5H₂O.

The term "sodium bicarbonate" (i.e. baking soda) as used herein is intended to include, but is not limited to, a white crystalline salt made by the action of carbonate on sodium having the molecular formula NaHCO₃.

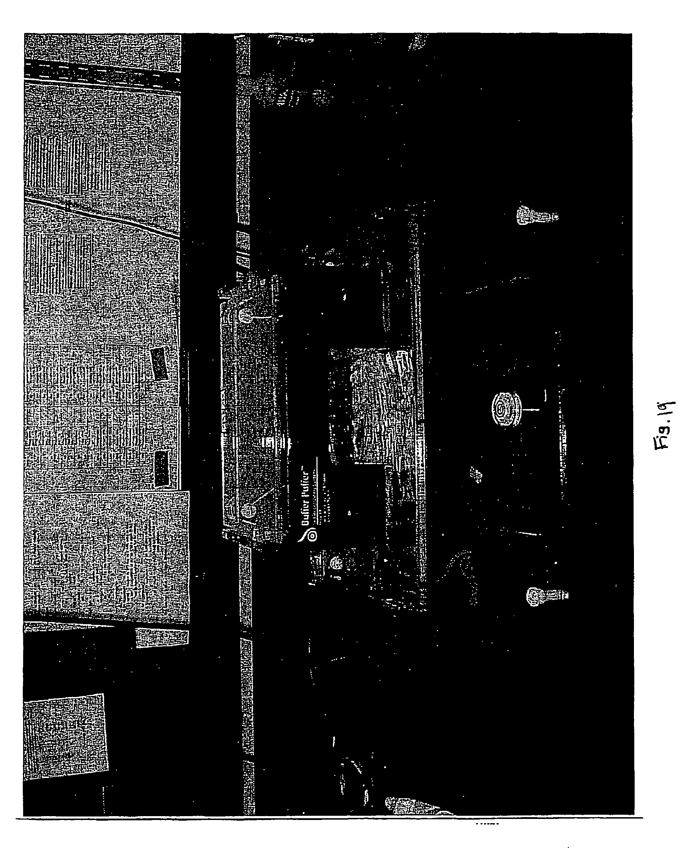

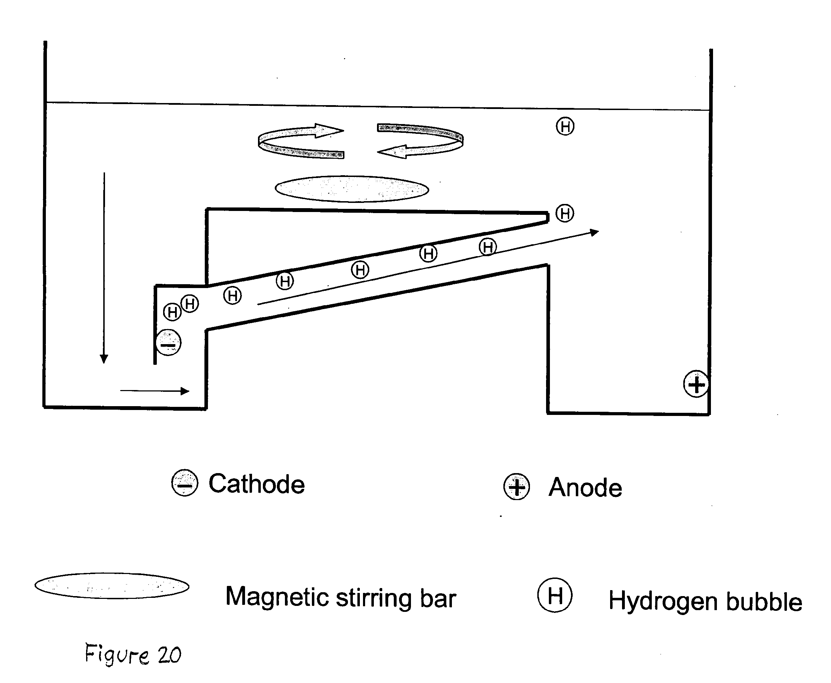

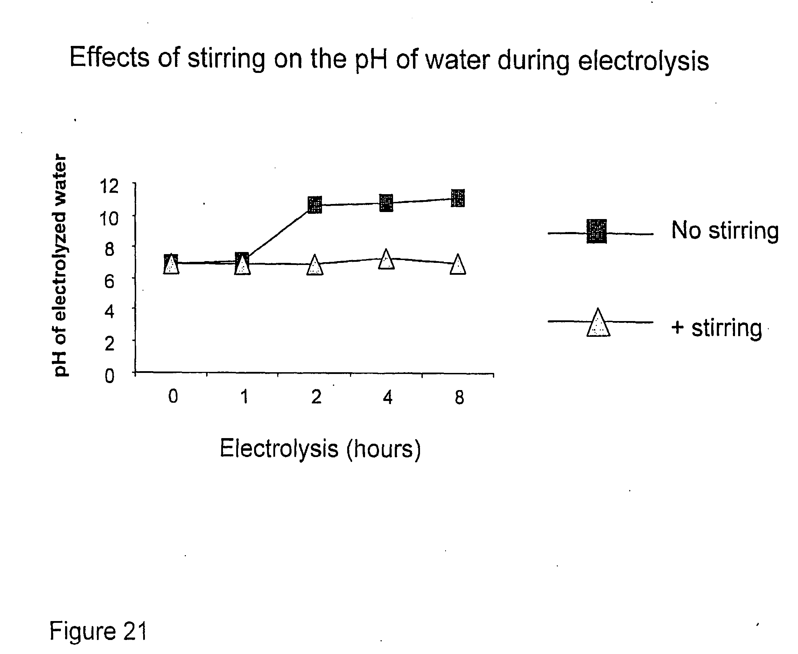

The term "electrolysis machine" as used herein is intended to include, but is not limited to, a standard machine capable of performing the electrolysis disclosed herein. Preferably, the machine is a gel electrophoresis machine such the Buffer Puffer™ Horizontal System (Figure 21, top machine in the picture, Buffer Puffer™ B3, 1 liter size; bottom machine in the picture, Buffer Puffer™ A5, 2.5 litter size), which is produced by Owl Separation Systems (Figure 19). As shown in the Figure 20, the Buffer Puffer™ self-recirculating electrophoresis bath from Owl Separation Systems is a way of recirculating the electrolysis water in an apparatus to prevent the formation of pH or ion gradients. The water from one end of the electrolysis bath travels through a connecting tube to the other end, allowing the electrolysis water to recirculate without the need for pumps, tubing, or other cumbersome accessories. Bubbles, of which major element is hydrogen, is used for at the end near the positive electrode provide the force to push the water back to the other end of the electrolysis bath. The dissolving process of hydrogen into the water is promoted during the traveling of hydrogen containing bubbles through the connecting tube. The directions provided with the systems can be used in conjunction with below described methodology.

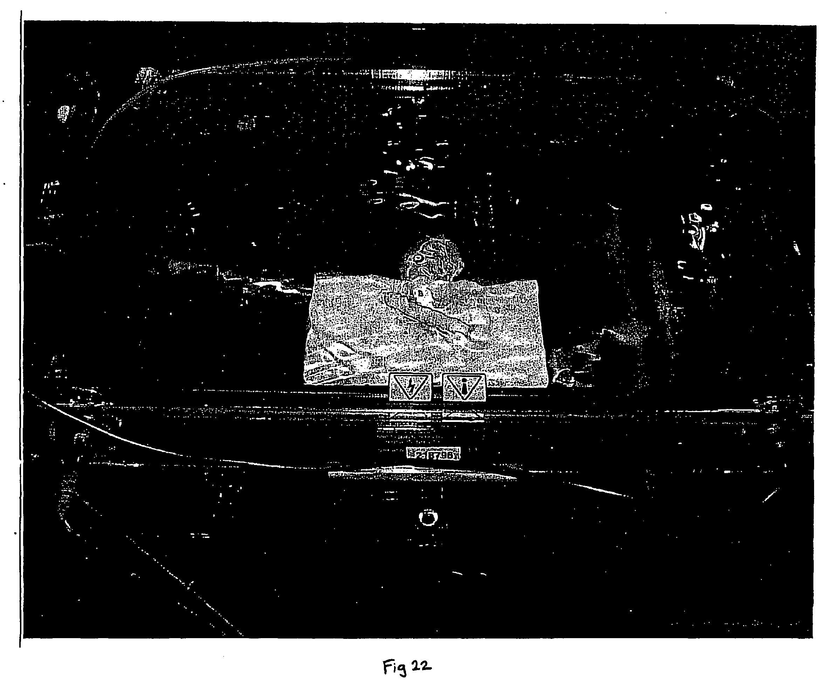

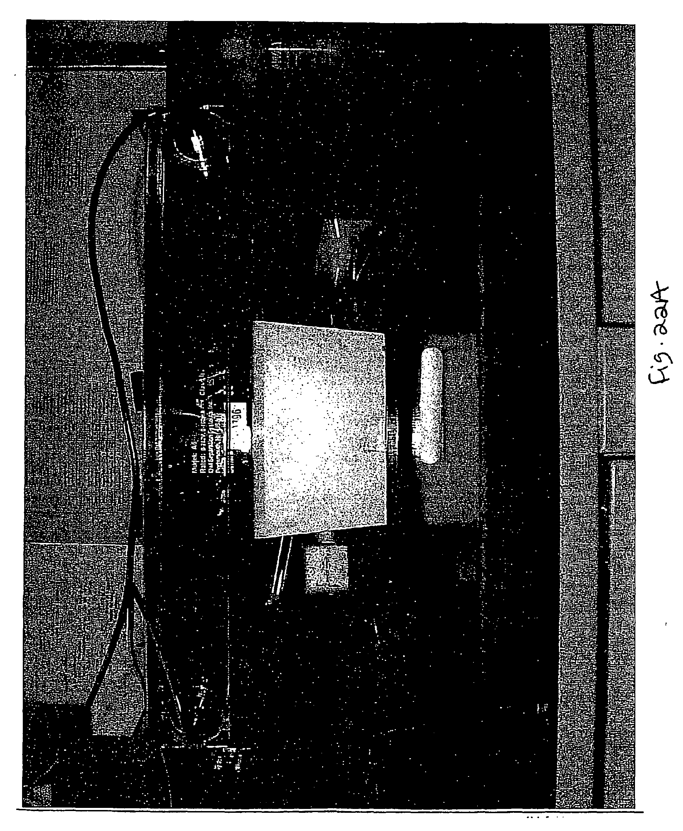

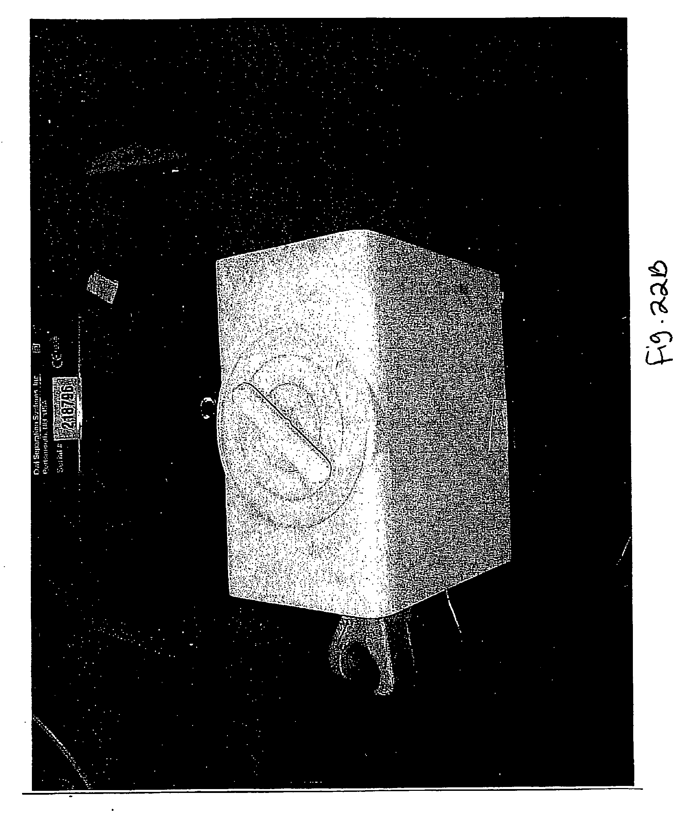

The IW of the present invention can be prepared via electrolysis within a diaphragm or diaphragmless electrolytic cell that is equipped with multiple electrodes, an oscillating stirrer, a self-recirculating connecting tube or a remote agitator can be used. The remote agitator equipped to the Buffer Puffer™ self-recirculating electrophoresis bath offers the generation of sufficiently efficient recirculation of water during the electrolysis process to prevent the formation of pH or ion gradients (Figure 20). The relevance of stirring in generation of neutral ionized water is shown in Figure 20. Using Buffer Puffer A5 system, the electrolysis of 0.1% NaCl solution (constant 30mA currency, room temperature) for more than two hours results in the increase of pH from 6.94 to 11.16. However, as the water was agitated by magnetic stirrer during the electrolysis of 0.1% NaCl solution (constant 30mA currency, room temperature), the pH of the water remained stable (0 hour, pH 6.94; 8 hour, pH 7.01). Preferably, the agitator is a magnetic stirrer. The remote magnetic stirrer can be any magnetic stirring device known to those of skill in the art. The stirrer can be placed not within, but underneath, the machine. The benefit of such placement is that the stirrer is still able to stir the water without contaminating the water. For example, a white Teflon®, a homopolymer of tetrafluoroethylene sold by DuPont, coated stirring bar, as shown in Figures 22-A, 22-B and 22-C, is placed in position underneath the machine. This configuration eliminates possible contamination of the water. The neutral ionized water is produced by dissolving a small amount at least one kind_of salt, e.g., CaCl₂, NaCl, Ca-lactate, or NaHCO₃ at concentration ranged between 0.05 - 0.2%, in tap water, distilled water, soft water, de-ionized water and RO (reverse osmosis membrane) water. Especially, the salt that does not contain CI (Chloride), such as Ca-lactate or NaHCO₃, can generate neutral ionized water without toxic derivative hypochlorite. Electrolysis is carried out by means of a direct current or pulsed current, while maintaining the voltage within a range of 1 to 30 V, and maintaining the current density within a range of 5 to 300 A/dm². The water is subjected to electrolysis at low water temperatures, at a range between 4 - 9°C, Hydrogen can also be injected into the water for elongating the shelf life of the water.

The ionized water disclosed herein can be used in preventing and treating diseases and conditions as disclosed above. Further, the ionized water can have anti-inflammatory, anti-bacterial, and other therapeutic effects upon administration to a patient. For example, the ionized water can inhibit the inducible nitric oxide synthase and tartrate resistant acid phosphatases that are involved in inflammation and bone resorption.

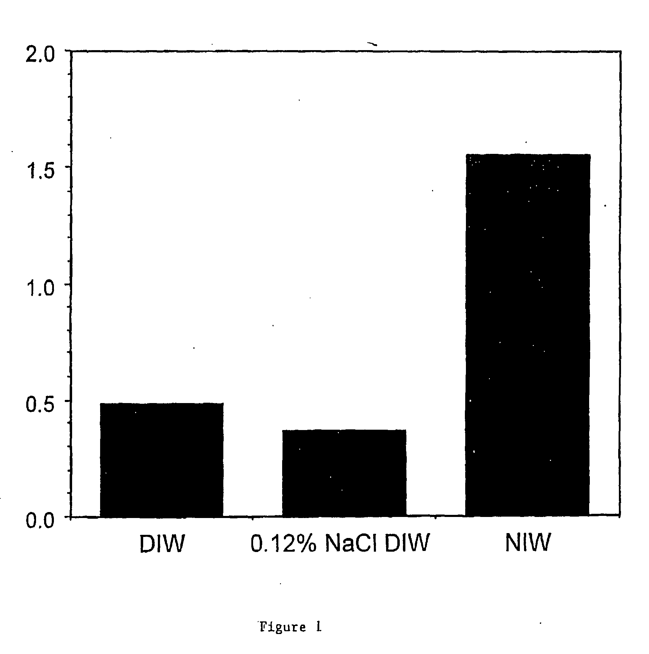

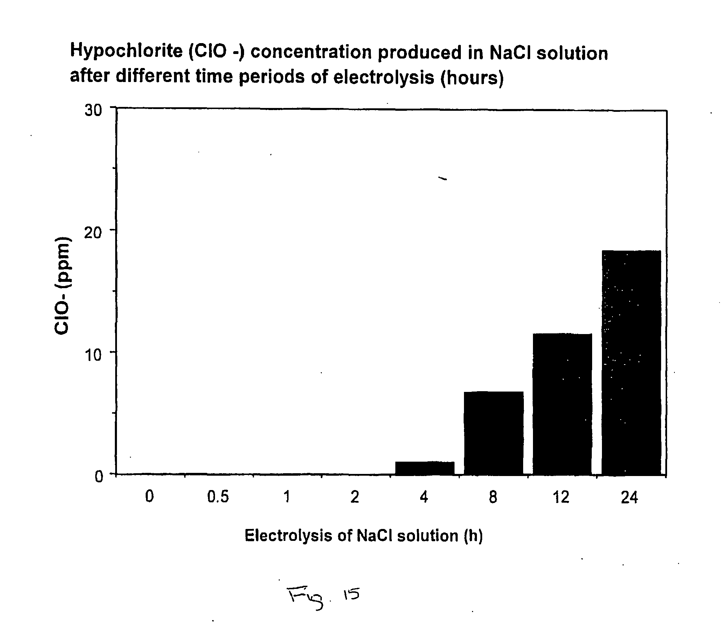

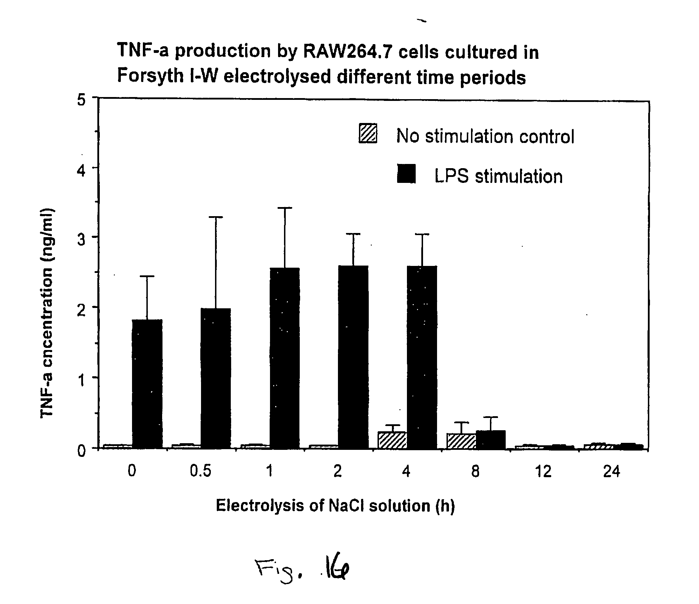

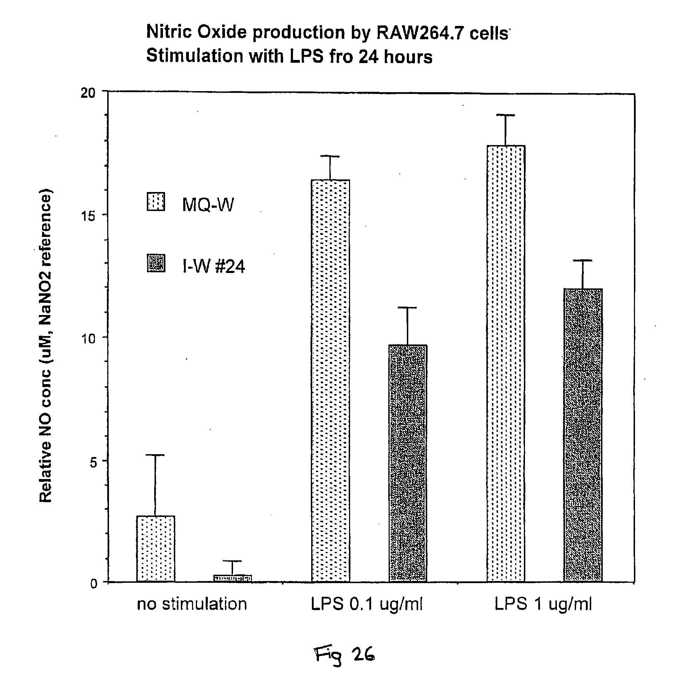

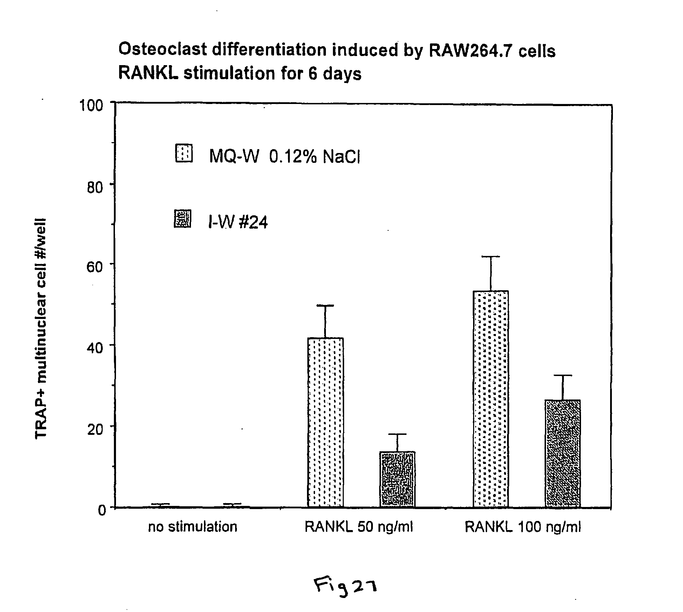

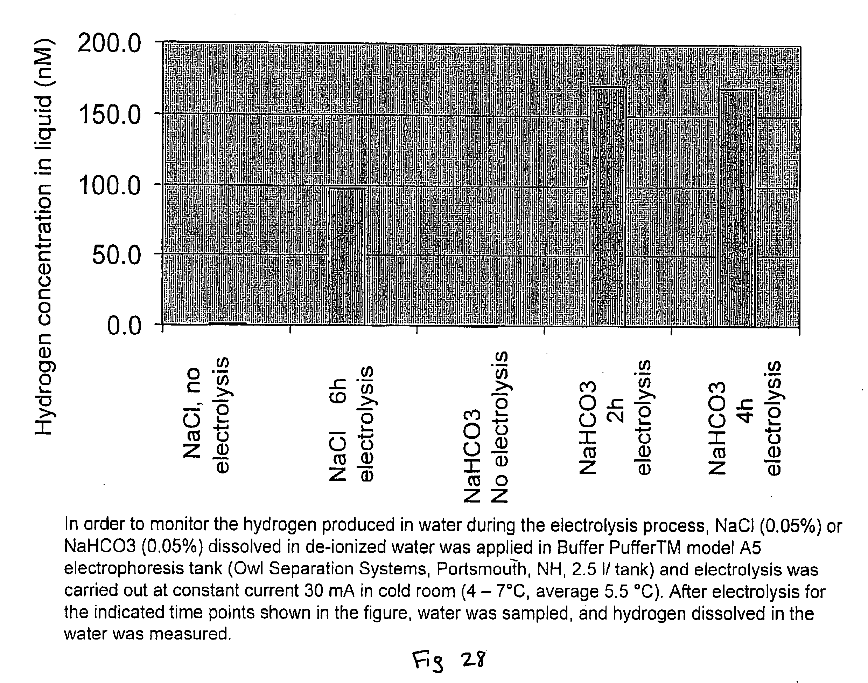

Chemical and biological properties of two different types of NIW is shown in the present invention; 1) NIW containing hypochlorite which is generated from NaCl containing solution (Figures 1-16, 23, 25B, 26, 27 and 30) and 2) NIW without hypochlorite which is generated from Calcium lactate or Sodium bicoarbonate (Figures, 17, 18, 24, 25A, 29). The biological properties of NIW of invention are attributed to the hydrogen accumulated in the NIW during the electrolysis process (Figure 1: NIW containing hypochlorite and Figure 28: NIW without hypochlorite). Thus, whether containing hypochlorite or not, the result is the same: both NIW elicit anti-inflammatory activities based on the common element present in the water, i.e. hydrogen, one of the most potent anti-oxidant.

As shown in Figure 1, the NIW contains greater than three times as much dissolved hydrogen as compared to control de-ionized water (DIW). More specifically, regular de-ionized water contained 0.5 ppm or less of dissolved hydrogen, whereas the NIW contained 1.5 ppm. Moreover, this elevated concentration of dissolved hydrogen was retained in the NIW at least two months after the NIW was generated using a electrolysis machine developed by Tokyo Techno (Tokyo, Japan).

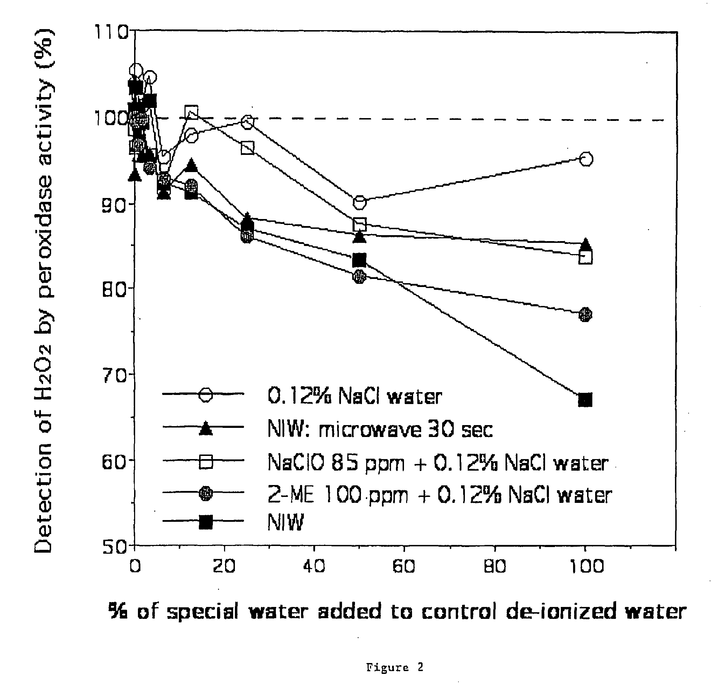

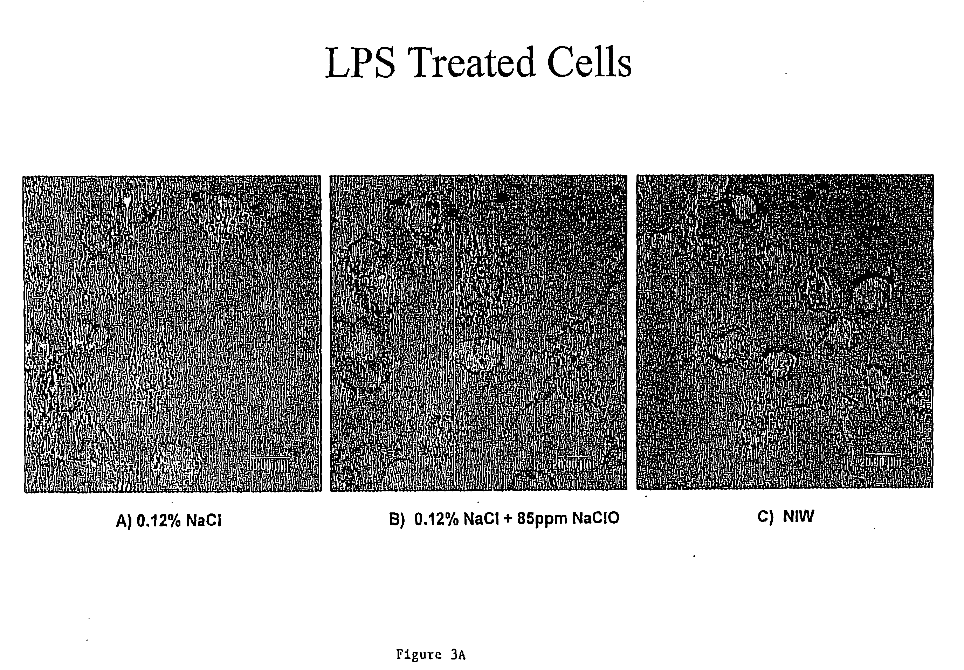

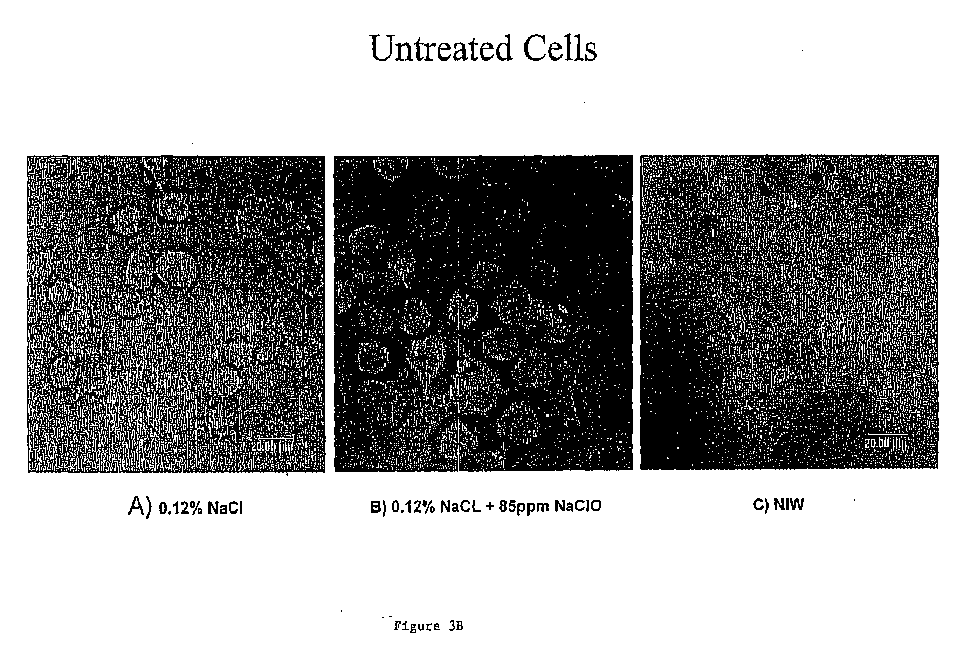

In addition, the NIW possesses anti-oxidant activity as demonstrated in Figures 2 and 3. More specifically, the NIW generated from NaCl solution showed anti-oxidant activity comparable to a control anti-oxidant, 2-ME, as compared to control unprocessed de-ionized waters. This effect was further seen in fluorescent microscopic analysis of RAW264.7 (mouse osteoclast precursor) cells, wherein NIW abrogated LPS-dependent ROS induction (see Example 2 below).

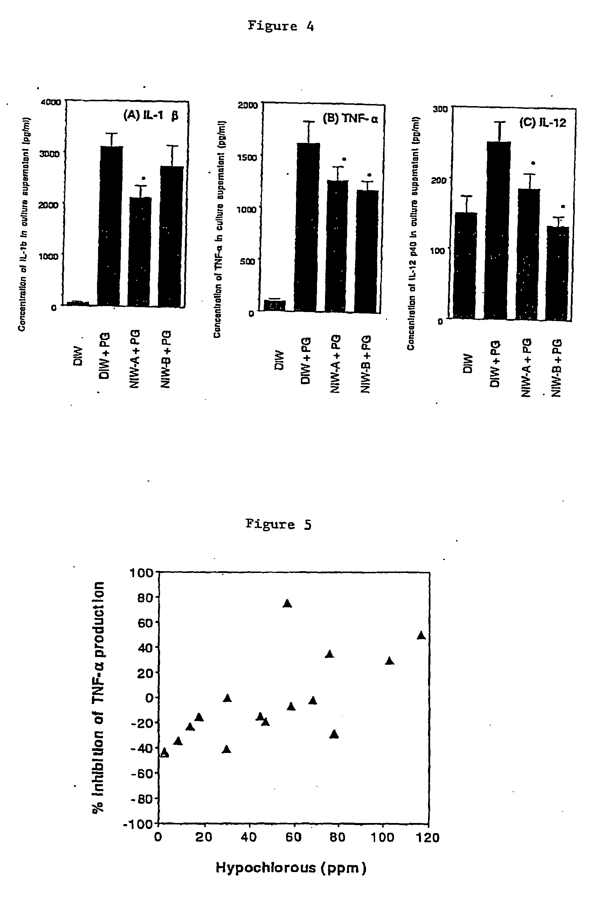

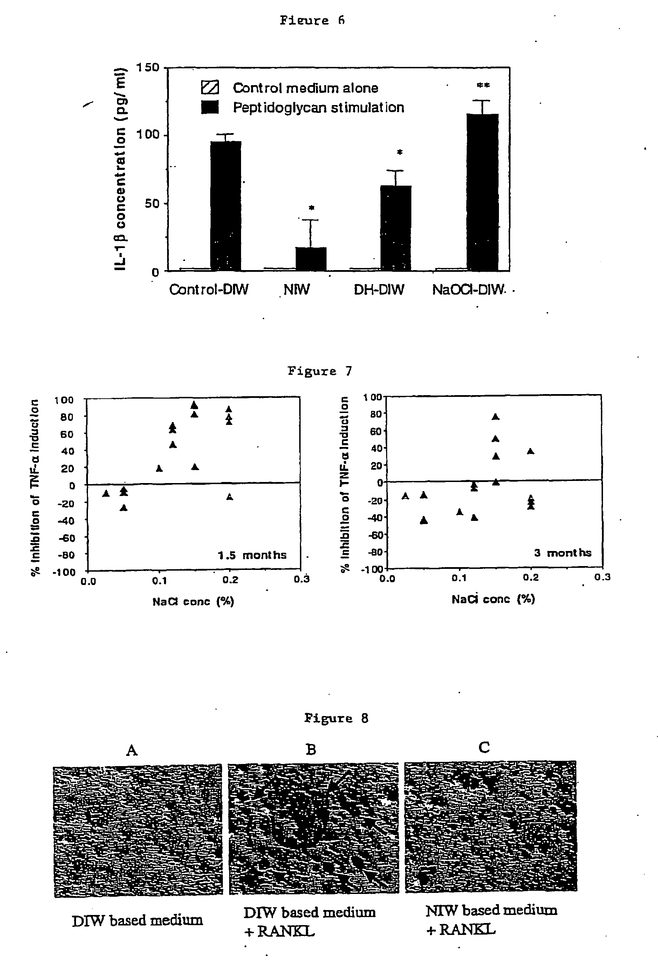

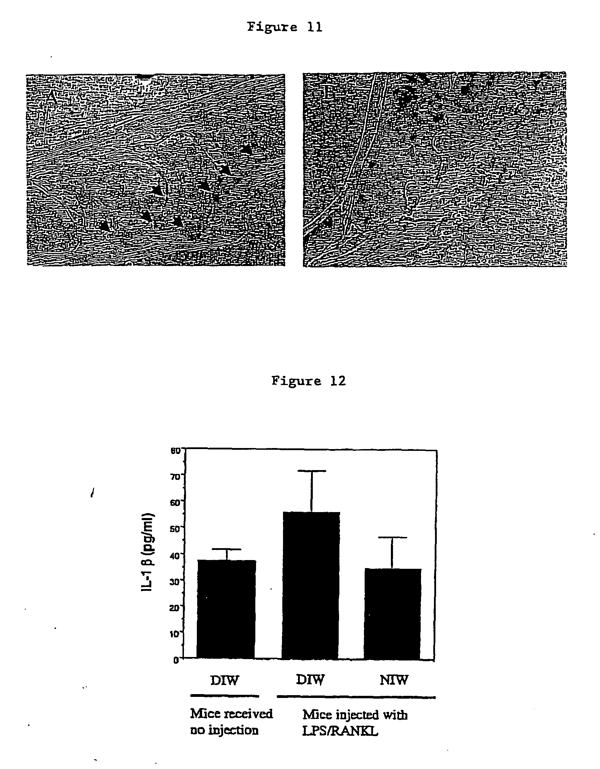

The NIW has numerous embodiments. According to one embodiment, treating and/or preventing inflammation. More specifically, utilizing NIW inhibited the production of proinflammatory cytokines including IL-1β, TNF-α and IL-12 (p40) by human peripheral blood mononuclear cells *in vitro* in response to mitogenic stimulation with peptidoglycan (Figures 4 and 6). In addition, NIW exhibited anti-inflammatory activity *in vivo* (Figure 12). Similar results were also demonstrated when LPS was used as the mitogen.

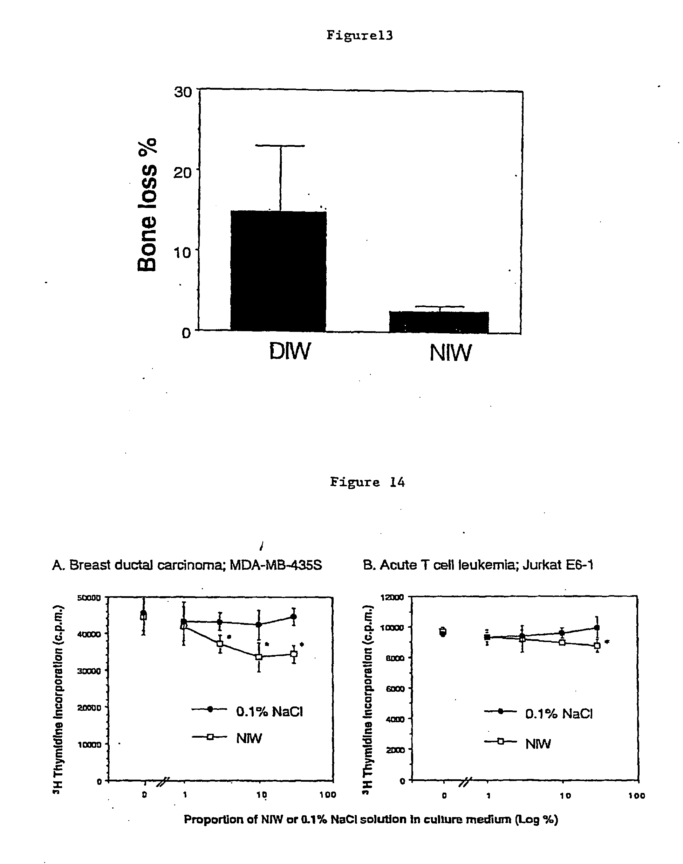

The NIW of the present invention inhibits RANKL/LPS-mediated osteoclast differentiation *in vitro* and *in vivo* (Figures 8-11). More specifically, NIW generated from NaCl solution significantly down-regulated bone resorption induced by RANKL/LPS injection into the mouse calvaria tissues (Figure 13).

NIW generated from NaCl solution suppresses the growth of two types of human cancer cell lines: MDA-MB-435S (Human breast ductal carcinoma) and Jurkat E6-1 (human acute T cell leukemia) (Figure 14).

Thus, NIW is useful in the treatment of various diseases and disorders, including cancer, osteoporosis, rheumatoid arthritis and periodontal disease. In addition, the NIW has various cosmetic and nutraceutical applications, e.g., in the treatment of psoriasis, acne and canker sores (recurrent minor aphthous ulcers).

In use, the ionized water is administered and dosed in accordance with good medical practice, taking into account the clinical condition of the individual patient, the site and method of administration, scheduling of administration, patient age, sex, body weight and other factors known to medical practitioners. The pharmaceutically "effective amount" for purposes herein is thus determined by such considerations as are known in the art. The amount must be effective to achieve improvement including but not limited to improved survival rate or more rapid recovery, or improvement or elimination of symptoms and other indicators as are selected as appropriate measures by those skilled in the art.

The ionized water of the present invention can be administered in various ways. It should be noted that it can be administered as the water alone or as an active ingredient in combination with pharmaceutically acceptable carriers, diluents, adjuvants and vehicles. The IW is preferably administered orally, but it can also be administered in other acceptable ways such as subcutaneously or parenterally including intravenous, intraarterial, intramuscular, intraperitoneally, and intranasal administration as well as intrathecal and infusion techniques. Implants of the compounds are also useful. The patient being treated is a warm-blooded animal and, in particular, mammals including man. The pharmaceutically acceptable carriers, diluents, adjuvants and vehicles as well as implant carriers generally refer to inert, non-toxic solid or liquid fillers, diluents or encapsulating material that do not react with the active ingredients.

It is noted that humans are treated generally longer than the mice or other experimental animals exemplified herein which treatment has a length proportional to the length of the disease process and drug effectiveness. The doses can be single doses or multiple doses over a period of several days, but single doses are preferred. The treatment generally has a length proportional to the length of the disease process and drug effectiveness and the patient species being treated.

When administering the IW of the present invention parenterally, it can be administered as a drink (e.g., soft drink or other formulation) or it can be formulated in a unit dosage injectable form (solution, suspension, or emulsion). Further, the IW of the present invention can be administered in topical applications. For example, the IW of the present invention can be applied directly to the skin, or incorporated into various cosmetics, powders, ointments, creams, oils, lotions, and the like. Additionally, the IW of the present invention can be added to nutraceuticals or other food supplements. The pharmaceutical formulations suitable for injection include sterile aqueous solutions or dispersions and sterile powders for reconstitution into sterile injectable solutions or dispersions. The carrier can be a solvent or dispersing medium containing, for example, water, ethanol, polyol (for example, glycerol, propylene glycol, liquid polyethylene glycol, and the like), suitable mixtures thereof, and vegetable oils.

Proper fluidity can be maintained, for example, by the use of a coating such as lecithin, by the maintenance of the required particle size in the case of dispersion and by the use of surfactants. Nonaqueous vehicles such a cottonseed oil, sesame oil, olive oil, soybean oil, corn oil, sunflower oil, or peanut oil and esters, such as isopropyl myristate, can also be used as solvent systems for compound compositions. Additionally, various additives, which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin. Any vehicle, diluent, or additive used would have to be compatible with the compounds.

A pharmacological formulation can be administered to the patient in an injectable formulation containing any compatible carrier, such as various vehicle, adjuvants, additives, and diluents; or the compounds utilized in the present invention can be administered parenterally to the patient in the form of slow-release subcutaneous implants or targeted delivery systems such as monoclonal antibodies, vectored delivery, iontophoretic, polymer matrices, liposomes, and microspheres. Examples of delivery systems include: 5,225,182; 5,169,383; 5,167,616; 4,959,217; 4,925,678; 4,487,603; 4,486,194; 4,447,233; 4,447,224; 4,439,196; and 4,475,196. Many other such implants, delivery systems, and modules are well known to those skilled in the art.

The ionized water of the present invention can be administered initially by intravenous injection to bring blood levels to a suitable level. The patient's levels are then maintained by an oral dosage form, although other forms of administration, dependent upon the patient's condition and as indicated above, can be used.

The invention is further described in detail by reference to the following experimental examples. These examples are provided for the purpose of illustration only, and are not intended to be limiting unless otherwise specified.

### EXAMPLES

### Materials and methods:

### Culture medium

For the culture of human peripheral blood mononuclear cells, human cancer cell lines and the mouse RAW264.7 cell line, culture mediums were prepared by dissolving α-MEM powder formula (Sigma) in either control 0.1% NaCl solution in de-ionized water (DIW, generated from tap water using Barnstead Nanopure Infinity system) or NIW. NIW was generated by the electrolysis of 0.1% NaCl solution in DIW using a NIW process machine made by Tokyo Techno (Tokyo, Japan). NIW was also generated by electrolysis of 0.05% - 0.2% CaCl₂, NaCl, Ca-lactate, or NaCHO₃ in DIW using the machine. The dissolved medium was sterilized by passing through a 0.2 µm pore size filter and supplemented with 10% FBS (fetal bovine serum), penicillin (100 U), streptomycin (100 µg/ml), L-glutamine (2 mM) and HEPES (0.01 M). The medium supplemented with 10% FBS plus penicillin/streptomycin, L-glutamine and HEPES is referred to herein as "complete medium."

### Measurement of hypocloric acid concentration in water.

DPD chlorine test kit (Sugiken inc. Tokyo Japan) was used to measure the concentration of hypochlorous acid (or hypochlorite) after modification of the protocol provided by the manufacturer.

Natural magnesium-based DH generation kit (Kasseisuisokun, Water Institute Inc., Tokyo, Japan) was utilized. Using this kit, DH (0.4 - 1.5 ppm) in DIW was generated.

### Example One for background purposes: NIW has a higher concentration of dissolved hydrogen than unprocessed waters

NIW generated from NaCl solution, control de-ionized unprocessed water (DIW) or DIW containing 0.12% NaCl was applied into extensively washed and air-dried glass tubes (8.5 ml/tube, Vacutainer tube, Becton Dickinson) at conventional atmosphere and tightly sealed with a rubber cap. After 2 days, the hydrogen concentration in the headspace (4.5 ml) of each tube was measure by a Kappa-3 reduction gas analyzer RGA-3/E001 (Trace Analytical). The head space air from the tubes containing NIW, DIW or DIW containing 0.12% NaCl showed that the hydrogen concentration of the NIW was 1.55 ppm as compared to the concentrations of the DIW controls, which were 0.48 ppm and 0.36 ppm, respectively (see Figure 1).

### Example Two for background purposes: The anti-oxidant effect of NIW generated from NaCl solution

A variety of water samples were serially diluted in control de-ionized unprocessed water (DIW), so that all the sample dilutions involved 0.005% H₂0₂. NIW was generated by electrolysis for 210 min in the presence of 0.12% NaCl, and contained 85 ppm NaClO. One sample of NIW (10 ml) was further treated with microwave exposure (at 1000 W for 30 seconds). NaClO 85 ppm or 2-mercaptoethanol at 100 ppm (2-ME, reductant/antioxidant) was dissolved in DIW containing 0.12% NaCl. All diluted water samples (200 µl/well in 96-well ELISA plates) were incubated at 37°C for 12 hours. After the incubation, 25 µl of I0x citrate buffer containing o-Phenylenediamine dihydrochloride (OPD, 20 mg/ml) and horse radish peroxidase (300 dilution of horse radish peroxidase conjugated antibody, Sigma A-8919) were mixed with the samples. The color development of OPD as a result of peroxidase activity was dependent on the concentration of H₂O₂ in the samples. The color was stopped after 10 min incubation by adding 2N H₂SO₄ (50 µl/well). As demonstrated in Figure 2, NIW reduced the H₂0₂ concentration in the samples in a manner similar to the anti-oxidant control, 2-ME.

In addition, the NIW demonstrated an anti-oxidant effect on the intracellular ROS induced by inflammatory stimulation. More specifically, RAW264.7 cells were incubated in 10% FBS containing α-MEM dissolved in a) 0.12% NaCl, b) 0.12% NaC1 + 85 ppm NaClO, or c) NIW. The cells were incubated in the presence or absence of E. *coli* LPS (1 µg/ml) for 24 hours. The cells were stained with Fluorescein-derivative ROS reacting reagent, 5-(and 6)-carboxy-2',7'-dichlorodihydrofluorescein diacetate (#C400, Molecular Probes). The C400 only reacts with ROS and develops fluorescence emission at a wavelength range of 517-527 nm. The staining pattern was analyzed by 0.3 µm sequential optical sectioning at x400 or x1000 magnification with a Leica™ TCS/SP-2 laser scan confocal microscope.

As indicated in Figure 3A, LPS treatment induced intracellular ROS by the RAW264.7 cells cultured in a) 0.12% NaCl and b) 0.12% NaCl+ 85 ppm NaClO. In contrast, the RAW264.7 cells incubated in NIW-based medium abrogated the LPS-dependent ROS induction. The ROS stained with C400 exhibited a green color. However, RAW264.7 cells cultured in the absence of LPS did not show intracellular ROS irrespective of the culture medium containing a) 0.12% NaCl, b) 0.12% NaCl+ 85 ppm NaClO or c) NIW. These results demonstrated that NIW can inhibit ROS induction by LPS stimulation, while NIW itself did not induce ROS induction.

### Example Three for background purposes: Inhibitory effect of NIW generated from NaCl solution on proinflammatory cytokine production by human peripheral blood monocytes in vitro

Human peripheral blood sampled from healthy volunteers was collected in heparinized collection tubes (Vacutainer, Becton Dickinson). Informed consent was obtained from each subject prior to inclusion in this study. After washing the collected blood with PBS (phosphate buffered saline), mononuclear cells were isolated by a gradient centrifuge using Histopaque™ (Sigma). The mononuclear cells (2 x 10⁵ cells/200µl/well) were cultured in 96-well tissue culture plates with complete α-MEM medium dissolved in either NIW generated from 0.12% NaCl solution or DIW in the presence or absence of mitogenic agents, peptidoglycan (PG) or lipopolysaccharide (LPS). Culture supernatants were harvested after incubation of the mononuclear cells for 24 - 48 hour in a CO₂ incubator at 37°C.

More specifically, human peripheral blood mononuclear cells in 96-well tissue culture plates were stimulated with 5 µg/ml of peptidoglycan (PG: *Staphylococcus aureus* origin, from Sigma) in NIW-based complete medium of α-MEM (25% NIW, 75% DIW) or DIW-based complete α-MEM (100% DIW). Two types of NIWs generated by different electrolysis conditions were tested (NIW-A, electrolysis at 7.0 Ampere, 12.1 Voltage for 120 min or NIW-B, electrolysis at 11.0 Ampere, 12.1 Voltage 90 min). After 24 hours of incubation, culture supernatants were collected from the 96-well plates and diluted in an equal volume of PBS containing 0.05% Tween 20 detergent (PBST). Commercially available ELISA kits for IL-1β, TNF-α and IL-12 (p40) were employed to measure the concentration of each proinflammatory cytokine in the culture supernatants.

As indicated in Figure 4, NIW inhibited the production of proinflammatory cytokines including IL-1β, TNF-α and IL-12 (p40) by human peripheral blood mononuclear cells in response to mitogenic stimulation with peptidoglycan. Similar results were demonstrated when LPS was used as the mitogen.

In addition, there was a lack of correlation between the concentration of hypochlorous acid in the NIW and NIW's inhibitory effects on the induction of proinflammatory cytokines. Since hypochlorous acid is considered to be a major active component in NIW, an examination was carried out to evaluate the relation between the concentration of hypochlorous acid in NIW and the inhibitory effect of NIW on peptidoglycan-mediated TNF-α production by the mouse osteoclast precursor cell line, RAW264.7.

More specifically, the RAW264.7 cells (in 96-well tissue culture plates) were stimulated with 5 µg/ml of peptidoglycan (*Staphylococcus aureus* origin, from Sigma) in NIW-based α-MEM (25% NIW, 75% DIW) supplemented with 10% FBS. NIW containing fifteen different concentrations of hypochlorous acid were compared with respect to their effect on the production of TNF-α by the RAW264.7 cells. After incubation for 24 hours, culture supernatants were collected and diluted in equal volumes of PBS containing 0.05% Tween 20 detergent (PBST). A commercially available ELISA kit for TNF-α was employed.

As indicated in Figure 5, there was no correlation between the inhibitory effect of NIW on TNF-α production and the concentration of hypochlorous acid in the NIW. The correlation coefficient, R= 0.639 (N=15), between the two parameters indicates that no relationship exists between hypochlorite concentration in NIW and its anti-inflammatory effects.

Next, the effects of NIW generated from 0.12% NaCl solution or water containing DH (dissolved hydrogen) on proinflammatory cytokine, IL-1β, production by the RAW264.7 cells *in vitro* was studied. Briefly, mouse RAW264.7 cells in 96-well tissue culture plates were stimulated with 5 µg/ml of peptidoglycan (*Staphylococcus aureus* origin, from Sigma) in NIW-based complete medium of α-MEM (25% NIW, 75% DIW) or DIW-based α-MEM (100% DIW). Hydrogen was dissolved in DIW for two days (DH-DIW) or NaCIO (100 ppm) was additionally added to DIW. All media used in this assay were supplemented with FBS (10%). After 24 hours of incubation, culture supernatants were collected from the 96-well plates and diluted in an equal volume of PBS containing 0.05% Tween 20 detergent (PBST). A commercially available ELISA kit for IL-1β was employed to measure the concentration of IL-1β in the culture supernatants.

As indicated in Figure 6, NIW inhibited the production of proinflammatory cytokine, IL-1β, by the mouse monocyte cell line RAW264.7 in response to mitogenic stimulation with peptidoglycan. Similar results were demonstrated when LPS was used as the mitogen. DH-water also inhibited the induction of IL-1β by the RAW264.7 cells, whereas sodium hypochlorite (NaClO) water augmented the mitogenic activity of the peptidoglycan. The results from Figures 5 and 6 indicate that dissolved hydrogen in the NIW is the active component that elicits the anti-inflammatory effects on cultured cells.

### Example Four for background purposes: The stability of the anti-inflammatory effect of NIW generated from NaCl solution

In general, dissolved hydrogen in alkaline ionized water is very short lived. In order to examine the stability of NIW's anti-inflammatory effects, sixteen different lots of NIW were tested for their effects in inhibiting TNF-α production by peptidoglycan-stimulated RAW264.7 cells, after different periods of storage at 4°C (1.5 months vs. 3 months). More specifically, sixteen lots of NIW containing different concentrations of NaCl were stored for one and a half months or for three months at 4°C. Mouse RAW264.7 cells in 96-well tissue culture plates were then stimulated with 5 µg/ml of peptidoglycan (*Staphylococcus aureus* origin, from Sigma) in NIW-based medium of α-MEM (25% NIW, 75% DIW) using these stored NIW lots. Thus, each medium was freshly created from stored NIW at one and a half months or three months. All media used in this assay were supplemented with FBS (10%). After 24 hours of incubation, culture supernatants were collected from the 96-well plates and diluted in an equal volume of PBS containing 0.05% Tween 20 detergent (PBST). A commercially available ELISA kit for TNF-α was employed to measure the concentration of TNF-α in the culture supernatants.

As shown in Figure 7, 11 lots of NIW maintained their anti-inflammatory effects after one and a half months of storage. After three months of storage, 4 lots of NIW maintained the anti-inflammatory effects. The most stable lots were found in NIW generated in the presence of 0.15% NaCl.

### Example Five for background purposes: In vitro analysis of inhibitory effect of NIW generated from NaCl solution on osteoclast differentiation

To analyze the effect of NIW on osteoclast differentiation, cells from the RAW264.7 osteoclast precursor cell line were stimulated with the osteoclast differentiation factor, RANKL (receptor activator of NF-kappa B Ligand) in control DIW-based complete α-MEM medium or NIW-based complete α-MEM medium for 6 days. More specifically, the RAW264.7 cells in 96-well tissue culture plates were incubated in the presence or absence of recombinant sRANKL (Peprotech, 50 ng/ml). In this study, the powdered formula of culture medium (α -MEM) was dissolved in either control DIW or NIW generated from 0.12% NaCl solution and supplemented with FBS, antibiotics and L-glutamine, as shown in the Material and Methods described above. After incubation for 6 days, the RAW264.7 cells were fixed with formalin and stained for tartrate-resistant acid phosphatase (TRAP) according to the method previously published (Kawai, T. et al. and Valverde, P., et al.). TRAP- positive cells with three or more nuclei were counted as mature osteoclast cells..

As shown in Figure 8, TRAP-positive multinuclear cells representing mature osteoclasts were observed in the RAW264.7 cells that had been stimulated with RANKL in the control DIW medium (Figure 8B, big and round cells with multi-nuclei, indicated by arrows), but not in the NIW medium (Figure 8C).

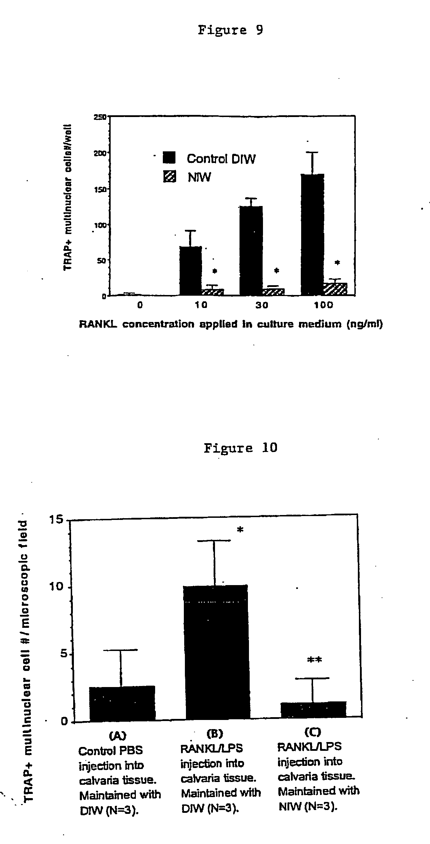

In addition, the RAW264.7 cells were cultured in 96-well tissue culture plates in the presence or absence of RANKL at various different concentrations, i.e., 10, 30 or 100 ng/ml. Control DIW-based- or NIW-based complete α-MEM were compared as to their effect on RANKL-mediated osteoclastogenesis. After incubation for six days, the RAW264.7 cells were fixed and stained for TRAP as described above. TRAP-positive cells with more than three nuclei in a well were counted under the microscope. The number of TRAP-positive multinuclear cells of each group is shown in Figure 9. As demonstrated by that figure, RANKL-dependent mature osteoclast differentiation was significantly down-regulated by NIW-based medium.

### Example Six for background purposes: In vivo evaluation of inhibitory effect of NIW generated from 0.12% NaCl solution on osteoclast differentiation using the mouse calvaria model

In order to evaluate NIW's effects on osteoclast differentiation induced *in vivo* by osteoclast induction factor RANKL and LPS, a mouse calvaria model was utilized (Li, L. et al.). In this study, C57/BL6 strain mice (male, 6 weeks old) received an injection of a mixture of LPS (100 µg/ml) and RANKL (10 µg/ml) dissolved in saline. The mixture was injected into the periosteum of the forehead of the mice (50 µl/animal), which were under anesthesia with ketamine and xylazine. From Day 0 when the mixture of LPS and RANKL was injected into the mice, NIW was given to the experimental group ad libitum , whereas the control groups of mice were maintained with regular DIW. More specifically, three groups of the mice (3 animals/group) were provided with either DIW (see Figure 10A or B) or NIW (Figure 10C) ad libitum during the total of 10 day experiment period. A mixture of RANKL and LPS was injected at Day 0 into the periosteum of forehead of groups B and C, whereas negative control group A did not receive any injections.

At Day 6, peripheral blood was collected from the mice and the concentration of IL-1β in the serum was measured by a commercially available ELISA kit (R&D Systems). In addition, surgically removed calvaria from the sacrificed animals of Groups B and C at day 10 were fixed with 5% formalin in saline and decalcified with EDTA treatment for 3 weeks. The decalcified calvaria was embedded into OCT compound (Tissue-Tek™, Sakura) and frozen at -70°C. Histochemical staining for TRAP, followed by methylene blue-based nuclear staining, identified mature osteoclast cells in the calvaria tissue. TRAP-positive cells with multi-nuclei were counted as mature osteoclast cells under the microscope (at x200 magnification).

As shown in Figure 10, RANKL and LPS injection into the periosteum of mouse calvaria induced TRAP-positive mature osteoclast cells in the tissue in 10 days in animals that were provided with DIW. In contrast, NIW-treated mice demonstrated a significantly lower number of TRAP-positive osteoclast cells in the calvaria tissue compared to the corresponding group provided with DIW. In addition, as shown in Figure 11, histological staining of the calvaria tissue demonstrated that TRAP-positive osteoclasts induced by the RANKL/LPS injection was significantly inhibited by NIW treatment (see Figure 11B). Moreover, as demonstrated in Figure 12, LPS/RANKL injection into the calvaria also increased the serum IL-1β concentration in the mice provided with DIW, whereas NIW treatment appeared to suppress the induction of IL-1β. These results indicate that NIW inhibits RANKL/LPS-mediated osteoclast differentiation and production of proinflammatory cytokine *in vivo.*

### Example Seven for background purposes: Evaluation of inhibitory effect of NIW generated from NaCl on in vivo bone resorption using a rat periodontal inflammation model

Although the calvaria model of Figures 10, 11, and 12 above demonstrated that NIW inhibits RANKL/LPS mediated osteoclast differentiation and induction of inflammatory factor, it was unclear if NIW interrupts bone loss that is induced by RANKL. Thus, it was conceivable that NIW only inhibits the formation of fresh osteoclasts induced by RANKL but does not interrupt the bone resorption activity by the previously differentiated authentic osteoclast cells. In order to address this question, a rat model of periodontal bone resorption was employed, which evaluated the physical bone loss induced by periodontal injection of RANKL/LPS. The rat periodontal inflammation model was utilized after modification of the previously published method (Kawai, T., et al.).

More specifically, Rowett strain rats (rnu/+ heterozygous normal females, 8 weeks old) received three palatal injections on the mesial of the first molar on the right and left sides (total of three sites on each side) of the maxilla. The left periodontal tissue received three injections of RANKL/LPS mixture, while the right side received three injections of control saline. The injection consisted of a mixture of LPS (500 µg/ml) and RANKL (10 µg/ml) dissolved in saline (50 µl/animal) or control saline alone. From Day 0 when the mixture of LPS and RANKL was injected into the rats, NIW was given to the experimental group ad libitum, whereas the control group of rats was provided with DIW for the 10 days of the total experiment period. Animals were sacrificed by CO₂ asphyxiation at Day 10, the jaws were defleshed, and periodontal bone resorption was measured on the palatal surface of the maxillary molars according to the method published previously. Periodontal bone resorption was compared between the RANKL/LPS-injected left side and the control saline-injected right side. The distances from the cementoenamel junction (CEJ) to the alveolar ledge (AL) of injected sites (upper left palatal side) and saline injected control sites (upper right palatal side) were measured using a reticule eyepiece at 25x magnification as previously described (Kawai, T., et al.). A total of five measurements were evaluated, including one point corresponding to the root axis of the second and third roots of the first molar, both roots of the second molar, and the first root of the third molar. The evaluation of bone loss was calculated and expressed as % bone loss = {(total CEJ-AL distance of 5 points of left experiment side) - (total CEJ-AL distance of 5 points of right control side)}/(total CEJ-AL distance of 5 points of right control side) x 100.

As shown in Figure 13, RANKL/LPS injection caused approximately 15% bone loss of the rat periodontal tissue. However, the bone resorption induced by RANKL/LPS injection was significantly down-regulated by NIW treatment.

### Example Eight for background purposes: In vitro evaluation of NIW's effects on cancer cell growth

The cancer cell growth inhibitory effect of NIW generated from 0.12 % NaCl solution was studied using two different human cancer cell lines. More specifically, the human breast ductal carcinoma cancer cell line, MDA-MB-435, and the human acute T cell leukemia line, Jurkat E6-1, were incubated in DMEM/F12 medium supplemented with 10% FBS for 24 hours in the presence of [³H] thymidine (0.5 uCi/well). The DMEM/F12 medium was composed of varying proportions of NIW vs. DIW. After the cells were incubated in the presence of [³H] thymidine (0.5 uCi/well) for 24 hours, radioactivity incorporated in the cancer cells reflecting the magnitude of cancer cell growth, was determined by liquid scintillation spectrometry. As shown in Figure 14, NIW demonstrated growth inhibitory effects on the two human cancer cell lines. As compared to control DIW, NIW significantly suppressed the growth of the human cancer cell lines.

### Example Nine:

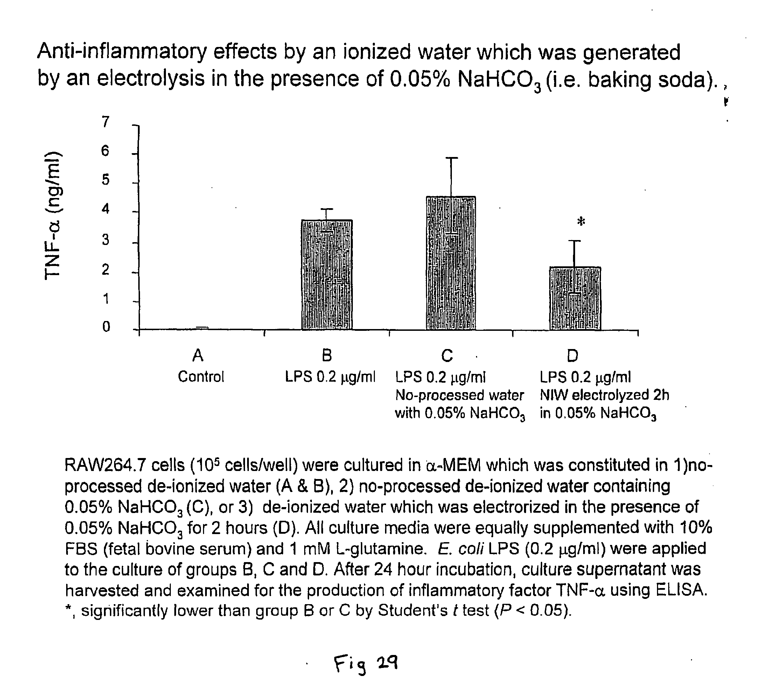

The figures show the results of several tests wherein RAW264.7 cells (10⁵ cells/well) were cultured in α-MEM that was constituted in non-processed de-ionized water (Figure 29, groups A and B), non-processed de-ionized water containing 0.05%NaHCO₃ (Figure 29, group C), or de-ionized water that was electrolyzed in the presence of 0.05%NaHCO₃ for two hours (Figure 29, group D). All of the culture media were supplemented with 10% FBS and 1 mM L-glutamine. E. coli LPS (0.2µg/ml) were applied to the culture of the groups B, C, and D. After a 24-hour incubation, culture supernatant was harvested and examined for the production of inflammatory factor TNF-α using ELISA. In order to monitor the hydrogen produced in water during the electrolysis process, NaCl (0.05%) for comparison only or NaHCO₃ (0.05%) dissolved in de-ionized water was applied in an electrophoresis tank and electrolysis was carried out at a constant current 30mA in a cold room (4-7 °C, average 5.5 °C). The results are shown in Figure 28. NIW was applied into extensively washed and air-dried glass tubes (8.5 ml/tube, Vacutainer tube, Becton Dickinson) at conventional atmosphere and tightly sealed with a rubber cap. After two days, the hydrogen concentration in the headspace (4.5 ml) of each tube was measure by a Kappa-3 reduction gas analyzer RGA-3/E001 (Trace Analytical). The head space air from the tubes containing NIW containing 0.05% NaCl or 0.05% NaHCO₃ showed that the hydrogen concentration of the 98nM (NaCl-IW, electrolyzed for six hours), 171 nM (NaHCO3-NIW electrolyzed for two hours), 168 nM (NaHCO3-NIW electrolyzed for four hours) as compared to the concentrations of the control non-treated 0.05% NaCl solution or non-treated NaHCO3 which were 0.9 nM or 0 nM, respectively (see Figure 28)

### Example Ten:

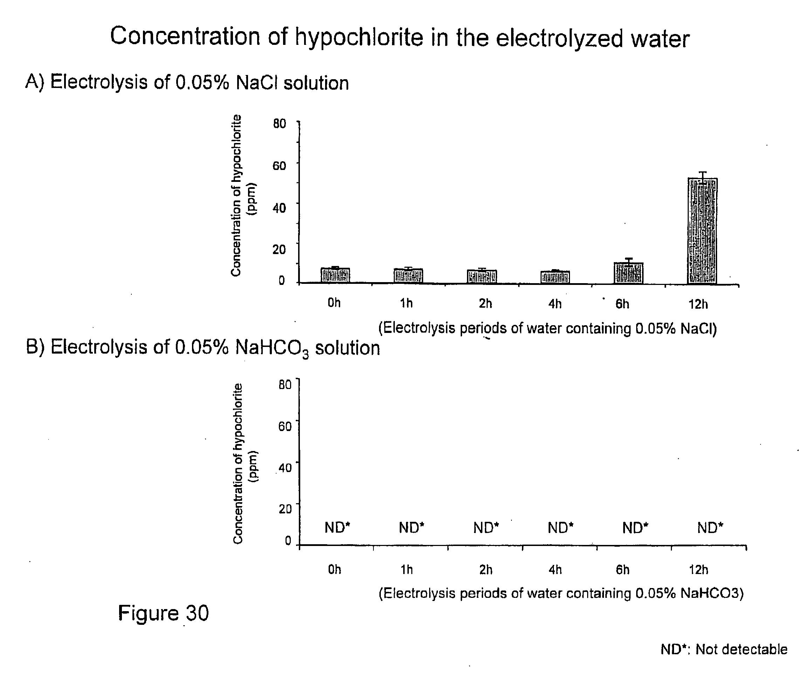

In order to monitor the hypochlorite concentration in the ionized water during the electrolysis process, NaCl (0.05%) for comparison only or NaHCO3 (0.05%) dissolved in de-ionized water was applied in Buffer Puffer™ model A5 electrophoresis tank (Owl Separation Systems, Portsmouth, NH, 2.5 I/ tank) and electrolysis was carried out at constant current 30 mA in cold room (4 - 7°C, average 5.5 °C) or on the conventional laboratory bench at room temperature (about 25 °C). After electrolysis for the indicated time points shown in the figure, water was sampled, and hypochlorite concentration was measured using SZK hypochlorite detection kit (Sugiken, Corp, Tokyo Japan) (Figure 30). The NIW generated in the presence of NaCl showed the increased concentration of hypochlorite in the electrolyzed water in a time dependent manner (Figure 30 A). However, The NIW generated in the presence of NaHCO₃ did not show any detectable level of hypochlorite during the electrolysis period up to 12 hours (Figure 30 B).

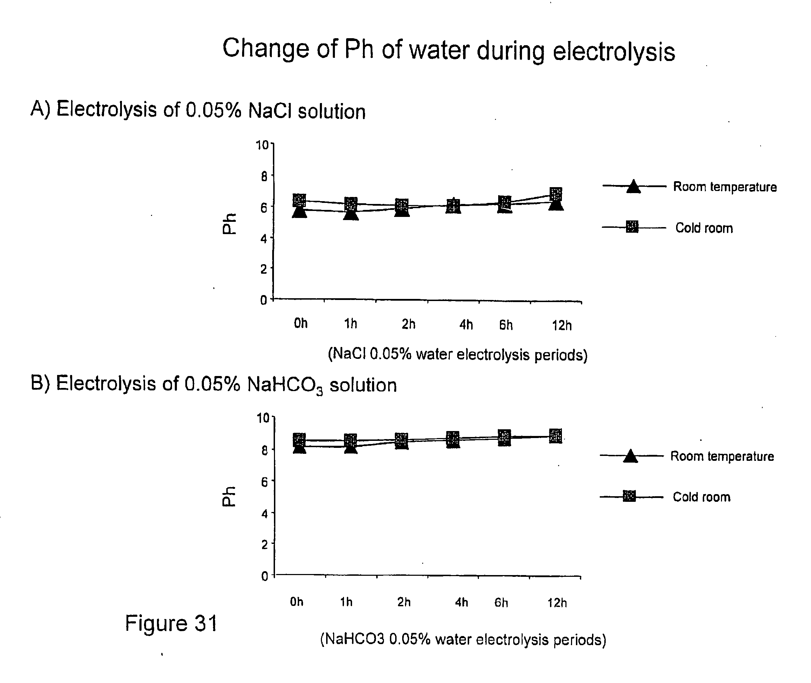

In order to monitor the change of pH of the water during the electrolysis process, NaCl (0.05%) or NaHCO3 (0.05%) dissolved in de-ionized water was applied in Buffer Puffer™ model A5 electrophoresis tank and electrolysis was carried out at constant current 30 mA in a cold room (4 - 7°C, average 5.5 °C) or on a conventional laboratory bench at room temperature (about 25 °C). After electrolysis for the indicated time points as shown in the figures, the water was sampled, and pH was measured using Accumet™ pH meter (Fisher Scientific)(Figure 31). The NIW generated in the presence of either NaCl or NaHCO₃ showed the stable pH during the electrolysis period up to 12 hours, irrespective of the temperature of the water (Figure 31A, NaCl-solution; Figure 31B, NaHCO₃-solution).

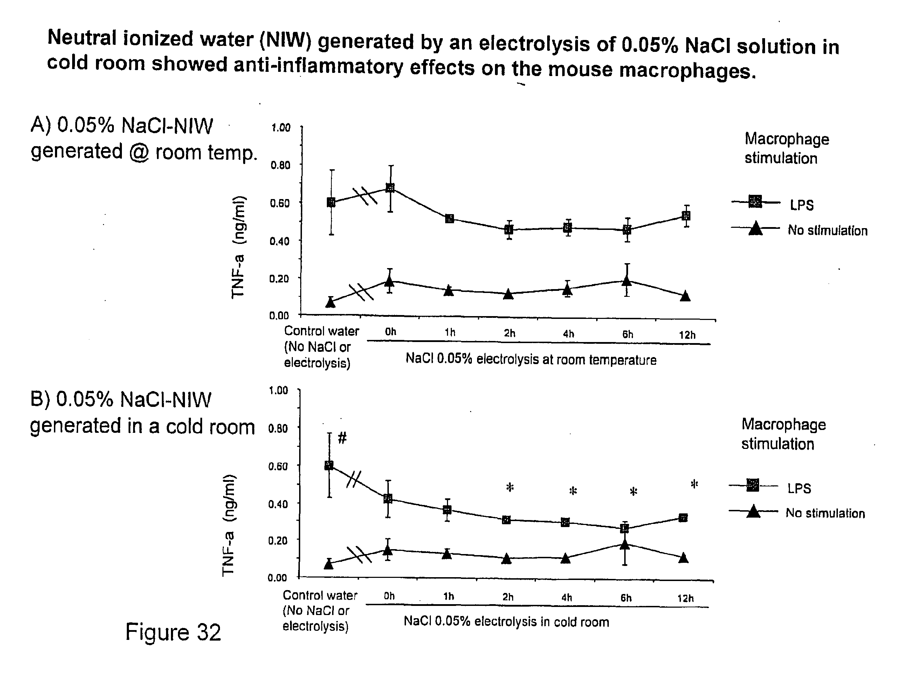

Macrophage cells isolated from C57BL6 mouse peritoneal cavity were cultured in α-MEM which was constituted in 1) non-processed de-ionized water without NaHCO3 (control), 2) non-processed de-ionized water containing 0.05% NaCl (0 h), or 3) de-ionized water which was electrolyzed in the presence of 0.05% NaCl for various periods at the room temperature (Figure 32A) or in a cold room (Figure 32B). After generation of each type of water, the resulting water, irrespective of electrolysis (time 0-12 hours), was kept at the room temperature or in a cold room until the macrophage culture assay. All culture media were equally supplemented with 10% FBS (fetal bovine serum) and 1 mM L-glutamine. *E. coli* LPS (2 µg/ml) were applied to the culture. After 24 hour incubation, culture supernatant was harvested and examined for the production of inflammatory factor TNF-α using ELISA. *, significantly lower than group # by Student's *t* test (P < 0.05). The NaCl-NIW generated in room temperature did not show a significant change of TNF-α expression by LPS-stimulated macrophages (Figure 32A). However, NaCl-NIW generated in a cold room, which was electrolyzed more than 2 hours, significantly suppressed the TNF-α expression by LPS-stimulated macrophages (Figure 32B).

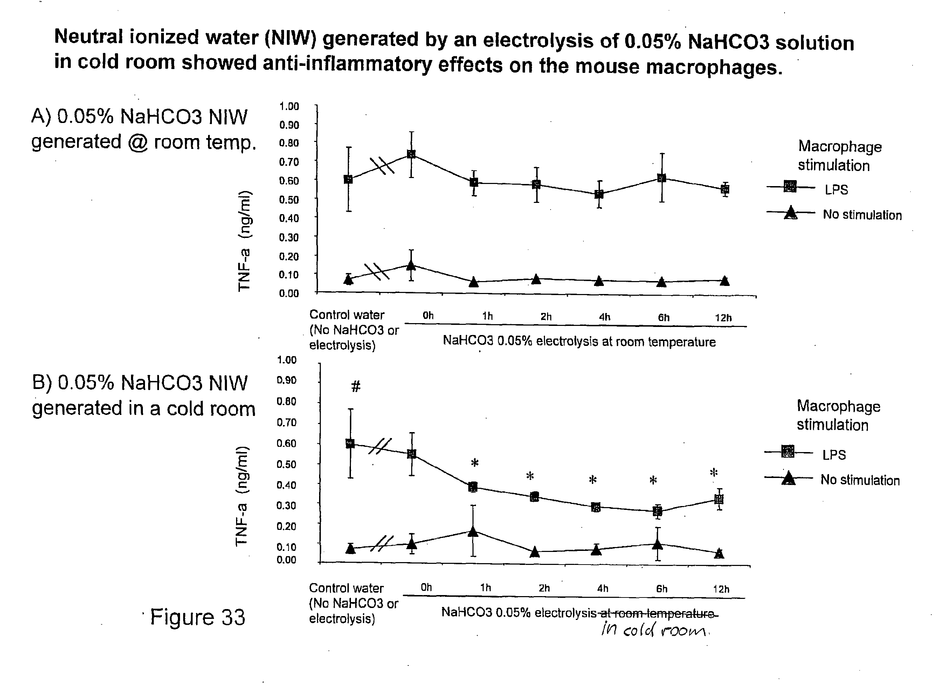

Mouse macrophage isolated from peritoneal cavity, similarly to Figure 32, were cultured in α-MEM which was constituted in 1) non-processed de-ionized water without NaHCO₃ (control), 2) non-processed de-ionized water containing 0.05% NaHCO₃ (0 hours), or 3) de-ionized water which was electrolyzed in the presence of 0.05% NaHCO₃ for various periods at the room temperature (Figure 33A) or in a cold room (Figure 33B). All culture media were equally supplemented with 10% FBS (fetal bovine serum) and 1 mM L-glutamine. *E. coli* LPS (2 µg/ml) were applied to the culture. After 24 hour incubation, culture supernatant was harvested and examined for the production of inflammatory factor TNF-α using ELISA. *, significantly lower than group # by Student's *t* test (P < 0.05). The Na HCO₃-NIW generated in room temperature did not show a significant change of TNF-α expression by LPS-stimulated macrophages (Figure 33A). However, Na HCO₃-NIW generated in a cold room, which was electrolyzed more than one hour, significantly suppressed the TNF-α expression by LPS-stimulated macrophages (Figure 33B).

### Example Eleven for background purposes: In vivo effects of NIW on the induction of colitis.

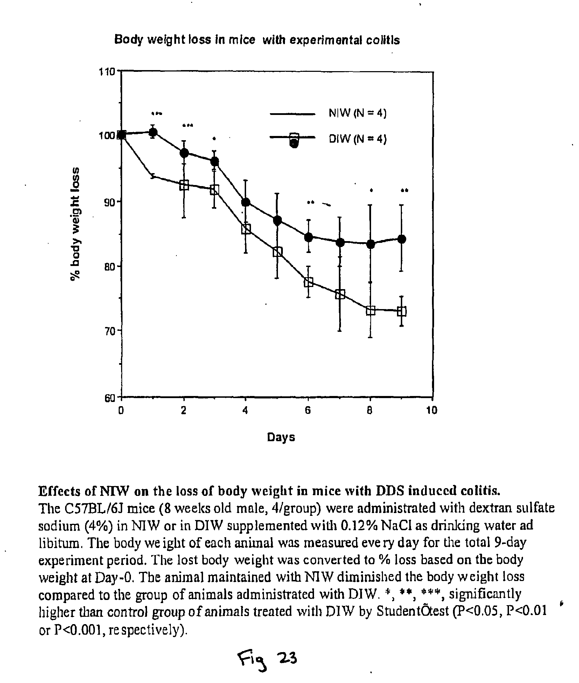

To determine the effects of the ionized water of the present invention on DDS induced colitis, C57BL/6J mice (8 weeks old male, 4/group) were administrated with dextran sulfate sodium (4%) in NIW or in DIW supplemented with 0.12% NaCl as drinking water ad libitum. The body weight of each animal was measured every day for the total 9-day experiment period (Figure 23). The lost body weight was converted to % loss based on the body weight at Day-0. The animal maintained with NIW diminished the body weight loss compared to the group of animals administrated with DIW (Figure 23). *, **, ***, significantly higher than control group of animals treated with DIW by Student' test (P<0.05, P<0.01 or P<0.001, respectively).

### Example Twelve for background purposes: In vivo preventive effects of NIW on the induction of acute hepatitis.

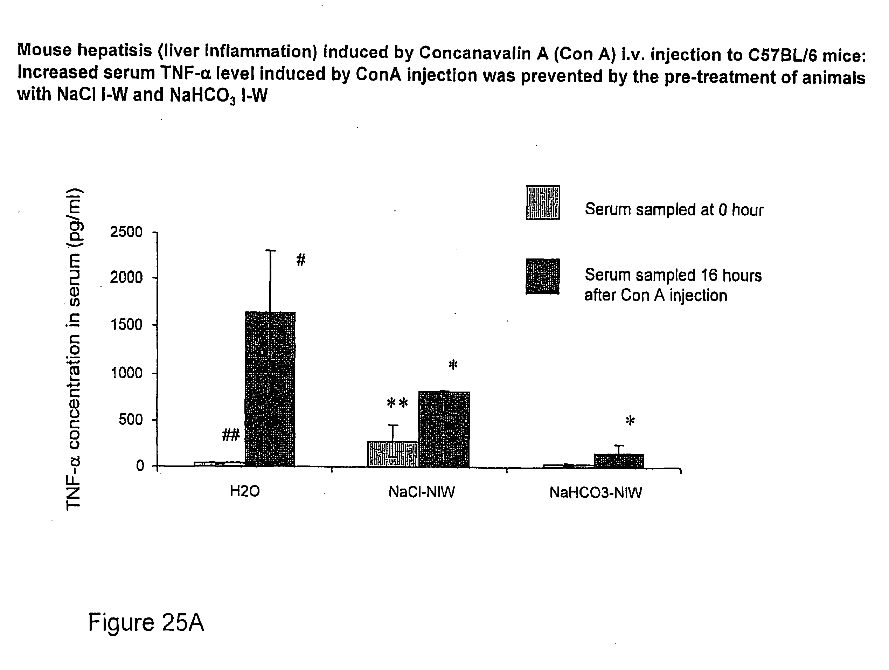

To determine the effects of the ionized water of the present invention on the prevention of onset of acute hepatitis, C57BU6 mice were treated with respective water, DIW (de-ionized water, control), NaCl-NIW or NaHCO3-NIW for three days in advance (Day-3) to the injection of Con A (15 mg/kg). NaCl-NIW or NaHCO3-NIW was generated by electrolysis of de-ionized water in the presence of 0.05% NaCl or 0.05% NaHCO3 for six hours in a cold room. Blood serum were isolated from animals before (0 hour) and after 16 hours from ConA injection. TNF-α concentration in the serum was measured by ELISA (Figure 25 A). *, significantly lower than group # by Student's *t* test (P < 0.05). **, significantly higher than group ## by Student's t test (P < 0.05). Increased serum TNF-α level induced by ConA injection was prevented by the pre-treatment of animals with NaCl I-W and NaHCO₃ I-W.

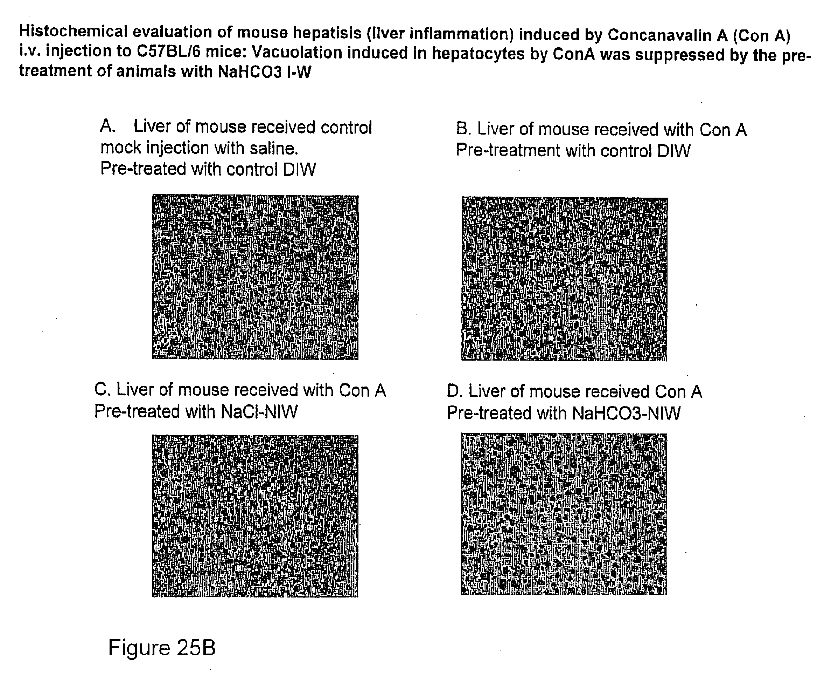

Figure 25B indicates the histological pictures of the livers of mice (C57BU6) induced with ConA-hepatitis. The mice were treated with respective water *ad libitum*; control DIW (Figure 25A & Figure 25B), NaCl-NIW (Figure 25C) or NaHCO₃-NIW (Figure 25D) for three days in advance to the injection of Con A (15 mg/kg). Sixteen hours after Con A injection, animals were sacrificed and livers were sampled. The sampled livers were sectioned by a cryostat (8 µm thickness) and stained with hematoxylin for histochemical analysis. Each section was examined using a microscope and image was captured by digital camera (x400 magnification). Figures 25B and 25C shows the pathogenic vacuolation formation, a sign of cell damage, in the hepatocytes. Pretreatment of mice with NaHCO₃-NIW inhibited the induction of such pathogenic vacuolation in the hepatocytes of mice that received ConA injection.

Throughout this application, author and year and patents by number reference various publications, including United States patents. Full citations for the publications are listed below.

### REFERENCES

Ghirardi, M. L., P. W. King, M. C. Posewitz, P. C. Maness, A. Fedorov, K. Kim, J. Cohen, K. Schulten, and M. Seibert. 2005. Approaches to developing biological H(2)-photoproducing organisms and processes. Biochem Soc Trans 33:70-2.
Grisham, M. B., D. Jourd'heuil, and D. A. Wink. 2000. Review article: chronic inflammation and reactive oxygen and nitrogen metabolism-implications in DNA damage and mutagenesis. Aliment Pharmacol Ther 14 Suppl 1:3-9.
Kawai, T., R. Eisen-Lev, M. Seki, J. W. Eastcott, M. E. Wilson, and M. A. Taubman. 2000. Requirement of B7 costimulation for Th1-mediated inflammatory bone resorption in experimental periodontal disease. J. Immunol. 164:2102-2109.
Lavrovsky, Y., B. Chatterjee, R. A. Clark, and A. K. Roy. 2000. Role of redox-regulated transcription factors in inflammation, aging and age-related diseases. Exp Gerontol 35:521-32.
Li, L., A. Khansari, L. Shapira, D. T. Graves, and S. Amar. 2002. Contribution of interleukin-11 and prostaglandin(s) in lipopolysaccharide-induced bone resorption in vivo. Infect Immun 70:3915-22.
Rutala, W. A., and D. J. Weber. 1997. Uses of inorganic hypochlorite (bleach) in health-care facilities. Clin Microbiol Rev 10:597-610.
Shirahata, S., S. Kabayama, M. Nakano, T. Miura, K. Kusumoto, M. Gotoh, H. Hayashi, K. Otsubo, S. Morisawa, and Y. Katakura. 1997. Electrolyzed-reduced water scavenges active oxygen species and protects DNA from oxidative damage. Biochem Biophys Res Commun 234:269-74.
Valverde, P., T. Kawai, and M. A. Taubman. 2002. Kaliotoxin decreases receptor activator of NFkB ligand (RANKL) expression in activated T cells in vitro and ameliorates local inflammatory bone resorption in experimental periodontal disease. J. Bone Min. Res. 17:S213.
Winterbourn, C. C. 2002. Biological reactivity and biomarkers of the neutrophil oxidant, hypochlorous acid. Toxicology 181-182:223-7.

## Claims

1. A method of forming hydrogen enriched hypochlorite free neutral ionized water comprising the steps of:
dissolving a non-hypochlorite generating salt in water at concentration of 0.05 to 0.2% and electrolyzing the water containing the dissolved salt at a temperature of between 4 and 9°C wherein said water is selected from the group consisting of tap water, distilled water, soft water, de-ionized water, and reverse osmosis membrane-formed water, wherein electrolysis is performed by maintaining a voltage range of 1 to 30V and a current density in a range of 5 to 300 A/dm².

2. The method according to claim 1, wherein said performing step includes electrolyzing by passing a current between at least two electrodes distantly placed in both ends of an electrolysis bath.

3. The method according to claim 1, wherein said performing step includes enabling hydrogen gas produced to travel through a tube to reach a distant end of the electrolysis bath where a cathode is placed, thereby increasing the dissolving process of hydrogen.

4. The method according to claim 1, further including continuously stirring the water during said performing step.

5. The method according to claim 4, wherein said stirring step includes stirring using a magnetic stirrer.

## Patentansprüche

1. Verfahren zur Herstellung von mit Wasserstoff angereichertem Hypochlorit-freiem neutralem ionisiertem Wasser umfassend die Schritte: Lösen eines nicht Hypochlorit erzeugenden Salzes in Wasser mit einer Konzentration von 0,05 bis 0,2% und Elektrolysieren des Wassers enthaltend das gelöste Salz bei einer Temperatur zwischen 4 und 9°C, wobei das Wasser ausgewählt ist aus der Gruppe bestehend aus Brauchwasser/Leitungswasser (tap water), destilliertes Wasser, weiches Waser, de-ionisiertes Wasser und durch Umkehrosmose hergestelltes Wasser, wobei die Elektrolyse bei Aufrechterhaltung einer Spannung in dem Bereich von 1 bis 30 Volt und einer Stromdichte in einem Bereich von 5 bis 300 A/dm² durchgeführt wird.

2. Verfahren nach Anspruch 1, wobei die durchzuführenden Schritte eine Elektrolyse umfassen, bei welcher ein Strom zwischen wenigstens zwei Elektroden, die voneinander beabstandet an beiden Enden des Elektrolysebades angeordnet sind, durchgeleitet wird.

3. Verfahren nach Anspruch 1, wobei die durchzuführenden Schritte umfassen, dass erzeugtem Wasserstoffgas ermöglicht wird, durch ein Rohr zu wandern, um ein beabstandetes Ende des Elektrolysebades, an welchem eine Kathode angeordnet ist, zu erreichen, um hierdurch den Lösungsvorgang von Wasserstoff zu verstärken.

4. Verfahren nach Anspruch 1, welches weiterhin ein kontinuierliches Rühren des Wassers während der Durchführung der Schritte umfasst.

5. Verfahren nach Anspruch 4, wobei das Rühren das Rühren mittels eines Magnetrührers umfasst.

## Revendications

1. Procédé de fabrication d'eau ionisée neutre sans hypochlorite, enrichie d'hydrogène comprenant les étapes suivantes :
- dissolution d'un sel ne générant pas d'hypochlorite dans de l'eau suivant une concentration de 0,05 - 0,2 % et électrolyse de l'eau contenant le sel dissout à une température comprise entre 4 et 9°C,
- l'eau étant choisie dans le groupe comprenant l'eau du robinet, l'eau distillée, l'eau douce, l'eau dé-ionisée et l'eau obtenue à travers une membrane d'osmose inverse,
procédé selon lequel
l'électrolyse est effectuée en maintenant une plage de tension de 1 à 30 V et une densité de courant dans une plage de 5 à 300 A/dm².

2. Procédé selon la revendication 1,
selon lequel
l'étape de réalisation comprend l'électrolyse par le passage d'un courant entre au moins deux électrodes placées à distance aux deux extrémités d'un bain d'électrolyse.

3. Procédé selon la revendication 1,
selon lequel
l'étape de réalisation consiste à autoriser la production d'hydrogène gazeux de cheminer à travers un tube et atteindre une extrémité distante du bain d'électrolyse à l'endroit d'une cathode, de façon à augmenter le procédé de dissolution d'oxygène.

4. Procédé selon la revendication 1,
selon lequel
en outre, on agite de manière continue l'eau au cours de l'étape de réalisation.

5. Procédé selon la revendication 5,
selon lequel
l'étape d'agitation consiste à agiter avec un agitateur magnétique.
